# EUROPÄISCHE PATENTSCHRIFT

(11) **EP 4 510 852 B1**
(45) Veröffentlichungstag und Bekanntmachung des Hinweises auf die Patenterteilung: **25.02.2026**
(21) Anmeldenummer: 23728778.4
(22) Anmeldetag: 26.05.2023
(51) Int. Cl.: A24F 40/46, A24F 40/20, A61M 15/06, A61M 11/04

(54) **EVAPORATOR ZUM EXTRAHIEREN UND FILTERN VON WIRKSTOFFEN AUS BIOLOGISCHEN MATERIALIEN DURCH ERHITZEN**
EVAPORATOR FOR EXTRACTING AND FILTERING ACTIVE INGREDIENTS FROM BIOLOGICAL MATERIALS BY HEATING
ÉVAPORATEUR POUR EXTRAIRE ET FILTRER DES PRINCIPES ACTIFS À PARTIR DE MATIÈRES BIOLOGIQUES PAR CHAUFFAGE

(30) Priorität: 27.05.2022 DE 102022113395
(43) Veröffentlichungstag der Anmeldung: 26.02.2025
(73) Patentinhaber: BG Braingate Technology GmbH, 51069 Köln (DE)
(72) Erfinder: SCHMITT, Fritz, 6585 Steinheim (LU)
(74) Vertreter: Angerhausen, Christoph
(86) Internationale Anmeldenummer: PCT/EP2023/064277
(87) Internationale Veröffentlichungsnummer: WO 2023/227789

(56) Entgegenhaltungen:
- WO-A1-2020/194286
- WO-A1-2021/105482
- WO-A1-2021/140463
- WO-A2-2021/025363
- US-A1- 2019 289 908

## Beschreibung

In medizinisch therapeutischer Anwendung hat der Inhalator/Aspirator einen festen Platz erobert, da nicht nur Wirkstoffe zum Inhalieren Verwendung findet, sondern je nach Indikation auch die Verordnung ganzer Cannabisblüten zur Anwendung kommt, die in einem Evaporator decarboxyliert werden, um die daraus extrahierten Wirkstoffe zu inhalieren. Dabei ist dem Patienten - besonders Nichtrauchern - keinesfalls die Schadbelastung durch Rauch bei klassischer Verbrennung zuzumuten.

Die WO 2021/105482 A1 beschreibt Evaporator zum Extrahieren und Filtern von Wirkstoffen aus biologischen Materialien durch Erhitzen, wobei in einer Heizkammer ein Wirkstoffvorläufer angeordnet ist, wobei der Wirkstoffvorläufer mit einer Heizmuffe umgeben ist, die verschiebbar auf dem Wirkstoffvorläufer sitzt, wobei die Länge des Wirkstoffvorläufers ein Mehrfaches der Breite der Heizmuffe beträgt, so dass eine Mehrzahl Teilbereiche des Wirkstoffvorläufers einzeln erhitzbar sind, so dass von dem Wirkstoffvorläufer bereitgestellter Wirkstoff zumindest bereichsweise rauchbar ist und/oder ein Aerosol, das bereichsweise von dem Wirkstoffvorläufer bereitgestellten Wirkstoff umfasst, erzeugbar ist.

Bei verbesserter Extraktion (Wirkungsgrad) mit dem Inhalator auf niedrigem Temperaturniveau bis max. 200°C ist eine weitaus geringere Schädigung des Lungensystems als bei Aufnahme nach Verbrennung nachgewiesen.

Auf dem weltweiten Markt werden viele unterschiedliche Geräte angeboten, die mit Heißluft, oder mit Heizplatten oder Glühdrähten, versuchen, Stoffe, Kräuter oder Flüssigkeiten bei einer genau definierten Temperatur so zu erhitzen, dass der gewünschten Wirkstoffe zur Inhalation zur Verfügung steht. Die meisten dieser Geräte arbeiten nach dem Herdplatten-Prinzip. Dabei werden die Kräuter auf einer erhitzten Fläche verteilt, in der Hoffnung, dass dabei eine Verdampfung der Inhaltsstoffe stattfindet. Diese Methode funktioniert nur unzureichend, da die in unmittelbarem Kontakt mit der erhitzten Fläche stehenden Substanzen wesentlich stärker erhitzt werden als darüberlegende Schichten, wodurch eine einigermaßen gleichmäßige Verdampfung der Inhaltsstoffe ausgeschlossen ist. Bei dem anderen Funktionsprinzip werden die Kräuter mit auf einen bestimmten Temperaturbereich erhitzter Luft durchströmt und dampfen - die notwendige Mindesttemperatur vorausgesetzt - ihre Inhaltsstoffe aus, welche dann nach Abkühlung der Luft inhaliert werden können.

Insbesondere bei Verwendung im medizinischen Bereich können die am Markt befindlichen Geräte keine Gerätesicherheit garantieren, wodurch alle Voraussetzungen für ein medizinisches Gerät fehlen. Beispielsweise können die Geräte können nicht sterilisiert werden. So ergeben sich Verbrennungsrückstände auf den Heizplatten oder "Grillrosten". Die Aufgabe der Erfindung besteht darin, ein Verfahren und Gerät nach den Vorgaben der EU-Verordnung 2017/745 (Medical Device Regulation, kurz MDR) zu schaffen, das Wirkstoffe insbesondere Cannabisblüten ohne eine exotherme Redoxreaktion auf 155°- 200°C portionsweise erhitzen kann. Bei Verwendung von Tabak kann aber auch eine Temperatur von max. 300°C vorgesehen sein.

Die Erfindung unterscheidet sich konstruktiv wesentlich von den am Markt befindlichen Inhalationsgeräten für Kräutermischungen, Cannabis und "Heat not Burn"-Geräten für Zigaretten.

Bei verbesserter Extraktion (Wirkungsgrad) mit dem Inhalator auf niedrigem Temperaturniveau ist eine weitaus geringere Schädigung des Lungensystems als bei Aufnahme nach Verbrennung nachgewiesen. Dabei ist dem Patienten - besonders Nichtrauchern - keinesfalls die Schadbelastung durch Rauch bei klassischer Verbrennung zuzumuten.

Das Trennen der Aerosole von den Schadstoffen und Kondensat ist ein wichtiger Bestanteil der Erfindung. Keines der am Markt befindlichen Geräten garantiert eine schadstoffreduzierte Inhalation. Die neue Erfindung garantiert eine deutlich geringere Schadstoffbelastung. Das wird dadurch erreicht, dass mehrere Systeme Schadstoffe filtern und Kondensat sammeln.

Die Erfindung bezieht sich auf "Zug-Inhalations-Evaporator" bzw. auf einen Evaporator solcher Zug-Inhalationsgeräte, welche einen intermittierenden, zugsynchronen Betrieb erlauben. Eine solche Betriebsart liegt vor, wenn die Wirkstoffportion nur während eines Zuges verdampft wird, während in Intervallen zwischen zwei Zügen im Wesentlichen keine Verdampfung erfolgt. Zug-Inhalations-Evaporatoren sind Inhalationsapparate, bei welchen das gebildete Dampf-Luft-Gemisch oder/und Kondensationsaerosol dem Benutzer wie bei einer Zigarette in zwei Schritten zugeführt werden kann, nämlich zuerst als Zug in die Mundhöhle (erster Schritt) und nach Absetzen des Evaporators in Form einer daran anschließenden Lungeninhalation (zweiter Schritt). Das Zugvolumen kann dabei individuell variieren, zwischen etwa 20 bis 80 mL. Im Gegensatz dazu erfolgt bei "klassischen Inhalationsgeräten" die Zufuhr des gebildete Dampf-Luft-Gemisches oder/und Kondensationsaerosols in einem einzigen Schritt als direkte Lungeninhalation. Es kann aber auch vorgesehen sein, dass das erzeugte Wirkstoffaerosol in nur einem Inhalationsvorgang verabreicht werden kann.

Klassische Inhalationsgeräte oder Evaporatoren weisen im Vergleich zu Zug-Evaporatoren einen deutlich höheren Luftdurchsatz von ca. 100 bis 750 mL/s gegenüber ca. 10 bis 40 mL/s durch den Inhalator bzw. Evaporator auf. Der vergleichsweise geringe Luftdurchsatz führt bei Zug- Evaporatoren im Zusammenwirken mit einem hohen Anteil an Wirkstoffen oder/und Cannabiswirkstoffen zu einer Reihe von Problemen. Genau dieses Problem wird bei der neuen Erfindung dadurch gelöst, dass ein Mischungsverhältnis zwischen Wirkstoff und Luftvolumen immer gleichbleibt, weil nur beheizt und damit Wirkstoff erzeugt wird, wenn inhaliert wird und sich nur durch mehr Zug und/oder Inhalation auch mehr Wirkstoff bilden und mischen kann. Eine Überdosierung ist bei dem erfindungsgemäßen Evaporator ausgeschlossen.

Als ein Wirkstoff z.B. Cannabis als Wirkstoffvorläufer für CBD und/oder THC gelten im Rahmen der gegenständlichen Patentanmeldung jede wirkstoffhaltige Lösung, deren Massenanteil an oder in dem erzeugten Aerosol nur so hoch ist, wie die gesetzlichen Vorschriften es erlauben.

Ein gesetzlich vorgegebener Wirkstoff Anteil z.B. bei der Verwendung von Cannabis als Wirkstoffvorläufer hat eine Reihe von vorteilhaften Effekten. Bei Cannabis sind die Grenzen der Wirkstoffe genau definiert, bereits schon bei der Aufzucht von Pflanzen.

Bekannte Evaporatoren sind üblicherweise dazu eingerichtet, zu rauchende Zigaretten und/oder Behälter, in den Wirkstoff aufgenommen sein kann, möglichst gleichmäßig zu beheizen. Damit kann sich eine inhomogene Erwärmung und damit inhomogene Gemischbildung z.B. aus Wirkstoff und Luft und/oder inhomogene Bildung von wirkstoffhaltigen Aerosolen ergeben. Insbesondere wenn verschiedene Wirkstoffe vorgesehen sind, die beispielsweise unterschiedliche Verdampfungstemperaturen und/oder Temperaturen, bei denen Wirkstoff verdampft und/oder extrahiert sein oder werden kann, oder unterschiedliche Mengen aufweisen, kann bei bekannten Evaporatoren die Gemischbildung und/oder ein Aerosolbildung nicht völlig zufriedenstellend sein.

Es ist eine Aufgabe der vorliegenden Erfindung, diese Nachteile zu überwinden. Diese Aufgabe wird durch einen Evaporator mit den Merkmalen des Anspruchs 1 gelöst. Besonders vorteilhafte Ausführungsformen sind jeweils Gegenstand der abhängigen Unteransprüche.

Die Erfindung betrifft einen Evaporator zum Extrahieren und Filtern von Wirkstoffen aus biologischen Materialien durch Erhitzen, wobei in einer Heizkammer ein Wirkstoffvorläufer in Form eines Hohlzylinders, dessen Außenwände durchlässig für Aerosole sind, angeordnet ist, wobei der Wirkstoffvorläufer mit einer Heizmuffe umgeben ist, die verschiebbar auf dem Wirkstoffvorläufer sitzt, wobei die Länge des Wirkstoffvorläufers ein Mehrfaches der Breite der Heizmuffe beträgt, so dass eine Mehrzahl Teilbereiche des Wirkstoffvorläufers einzeln erhitzbar sind, so dass von dem Wirkstoffvorläufer bereitgestellter Wirkstoff zumindest bereichsweise rauchbar ist und/oder ein Aerosol, das bereichsweise von dem Wirkstoffvorläufer bereitgestellten Wirkstoff umfasst, erzeugbar ist.

Der Evaporator kann die Heizkammer aufweisen. Der Evaporator kann den Wirkstoffvorläufer aufweisen. Der Evaporator kann die Heizmuffe aufweisen.

Es kann vorgesehen sein, dass in mindestens zwei Bereichen des Wirkstoffvorläufers zwei unterschiedliche Wirkstoffe bereitgestellt sein können, wobei die unterschiedlichen Wirkstoffe sich z.B. in ihrer Zusammensetzung und/oder Menge unterscheiden können.

Mit dem Evaporator kann beispielsweise das Abrauchen einer Zigarette mit deutlich reduzierter Schadstoffaufnahme simuliert und/oder nachgebildet sein oder werden. Prinzipiell sind alle Wirkstoffe denkbar, die bei einem Rauchvorgang und/oder durch Erhitzen und/oder Erwärmen z.B. verdampfbar und/oder extrahierbar sind, beispielsweise Cannabis.

Es kann vorgesehen sein, dass die Breite der Heizmuffe ca. 5% bis 25 % der Länge des Wirkstoffvorläufers betragen kann. In einigen Ausführungsformen können ca. 4 bis 15, Teilbereiche des Wirkstoffvorläufers einzeln erhitzbar sein.

Der Evaporator kann ein Mundstück aufweisen. Das Mundstück kann dazu eingerichtet sein, ein oder das Aerosol aus dem Evaporator auszuführen und/oder abzugeben. Das Mundstück kann dazu eingerichtet sein, Luft aus dem Evaporator auszuführen und/oder abzugeben. Ein Benutzer kann über das Mundstück beispielsweise das Aerosol inhalieren, einatmen, und/oder empfangen.

Die Heizkammer kann doppelwandig mit einer Innenwand und einer Außenwand ausgebildet sein. Zwischen Innenwand und Außenwand kann ein Hohlraum gebildet sein. Es kann vorgesehen sein, dass die Heizkammer ein doppelwandiger Zylinder sein kann. Ein oder der Innendurchmesser der Heizkammer und/oder Heizkammer des doppelwandigen Zylinders kann mindestens das doppelte eines Durchmessers des Wirkstoffvorläufer betragen.

In dem von der Innenwand und der Außenwand der Heizkammer gebildeten Hohlraum kann ein Vakuum existieren und/oder vorliegen. Alternativ kann sich in dem Hohlraum ein Wirkstoff und/oder Kühlmittel befinden.

Eine oder die Innenwand der Heizkammer kann eine Nanobeschichtung aufweisen.

Der Evaporator kann an einem dem Mundstück zugewandten Bereich der Heizkammer kann ein Filter aufweisen. Der Filter kann in einigen Ausführungsformen eines oder mehrere aus Aktivkohle, Schwamm und/oder Nassfilter, statisch aufgeladene Metallwolle, Meltblown-Vlies oder Kombinationen dieser umfassen, oder daraus bestehen. Der Filter kann ein Molekularsieb sein oder aufweisen.

Der Filter kann mehreren Segmenten aufweisen und/oder aus mehreren Segmenten bestehen. Damit können in einigen Ausführungsformen verschiedene Schadstoffe gefiltert sein oder werden. Es kann vorgesehen sein, dass jedes Segment für das Filtern eines spezifischen oder bestimmten Schadstoffs eingerichtet und/oder verwendet sein oder werden kann.

Der Evaporator kann an einem dem Mundstück zugewandten Bereich einen Superabsorber aufweisen, der dazu eingerichtet sein kann, Kondensat zu sammeln. Alternativ oder zusätzlich kann der Filter einen oder den Superabsorber umfassen.

Der Evaporator kann eine Leitung aufweisen, die dazu eingerichtet sein kann, Außenluft von einer Lufteintrittsöffnung des Evaporators in das Mundstück des Evaporators und/oder in die Heizkammer zu leiten. Es kann vorgesehen sein, dass die Leitung in der Heizkammer angeordnet ist und/oder zumindest teilweise in der oder durch die Heizkammer verlaufen kann. Es kann vorgesehen sein, dass die Leitung in einem oder dem von der Innenwand und der Außenwand der Heizkammer gebildeten Hohlraum angeordnet ist, und/oder zumindest teilweise in dem oder durch den Hohlraum verlaufen kann. Die Leitung kann die Lufteintrittsöffnung fluidisch mit der Heizkammer verbinden. Alternativ oder zusätzlich kann die Leitung kann die Lufteintrittsöffnung fluidisch mit dem Mundstück verbinden.

Eine Außenwand des Evaporators kann mit thermo-chromatischem Lack beschichtet sein. Der thermo-chromatische Lack kann dazu eingerichtet sein, temperaturabhängig seine Farbe zu ändern. Damit kann ein Benutzer erkennen, an welcher Position sich die Heizmuffe befindet, da z.B. dort lokal in der Heizkammer eine höhere Temperatur vorliegen und entsprechend lokal der thermo-chromatische Lack eine verschiedene Farbe haben kann.

Der Evaporator kann ein Gehäuse aufweisen, das aus einem magnetischen Drahtgitter bestehen oder ein magnetisches Drahtgitter aufweisen kann. Das magnetische Drahtgitter kann flächig mit Kunststoff beschichtet sein. Der Kunststoff kann, wenn Strom durch Drähte des Drahtgitters fließt, die stromleitende Drähte gegeneinander isolieren.

Der Evaporator kann mindestens ein an oder bei einer Oberfläche des Gehäuses angeordnetes Schaltelement aufweisen. Das Schaltelement kann induktiv mit mindestens einem Draht und/oder dem magnetischen Drahtgitter gekoppelt sein. Beispielsweise kann dadurch ermöglicht sein oder werden, dass ein magnetisches Schaltelemete auf der Oberfläche an einem beliebigen Punkt aufgesetzt werden kann. Das magnetische Schaltelement kann z.B. über Induktion Schaltvorgänge erzeugen. Ein Schaltvorgang kann z.B. ein Steuer Befehl für eine Steuerung des Evaporators sein oder aufweisen. Ein Schaltvorgang kann z.B. eine Anweisung umfassen, dass ein Rauchvorgang initiiert, und/oder die Heizmuffe aktiviert und/oder angesteuert sein oder werden kann. Ein Schaltvorgang ein Initiieren eines Rauchvorgangs, und/oder ein Aktivieren der Heizmuffe umfassen.

Der Wirkstoffvorläufer kann eine Aufnahme bilden. Die Aufnahme kann eine zylinderförmige Aufnahme sein. In der Aufnahme kann beispielsweise einer oder mehrere Wirkstoffe aufgenommen sein. Der Wirkstoffvorläufer kann derart dimensioniert sein, dass er die mindestens eine Länge von 8 cm, bevorzugt mindestens 8,4 cm, und einen Durchmesser von mindestens 0,6 cm, bevorzugt mindestens 0,8 cm, aufweisen kann. Es kann vorgesehen sein, dass der Wirkstoffvorläufer derart dimensioniert sein kann, um eine Zigarette komplett aufzunehmen. Es kann vorgesehen sein, dass der Wirkstoffvorläufer derart dimensioniert sein kann, dass eine Zigarette komplett in dem Wirkstoffvorläufer aufgenommen sein kann. Die Zigarette kann eine handelsübliche Zigarette sein, und/oder Abmessungen einer handelsüblichen Zigarette haben. Die Zigarette kann einen oder mehrere Wirkstoffe enthalten.

In einigen Ausführungsformen kann alternativ oder zusätzlich der Wirkstoffvorläufer eine Zigarette sein oder aufweisen.

Es kann vorgesehen sein, dass der Evaporator zum Rauchen einer in dem Wirkstoffvorläufer aufgenommenen Zigarette eingerichtet sein kann, und/oder zum Rauchen einer in dem Wirkstoffvorläufer aufgenommenen Zigarette dienen und/oder verwendet werden kann.

Der Wirkstoffvorläufer und/oder die Aufnahme kann einen zylinderförmigen Drahtkorb aufweisen. Der Drahtkorb kann in mehrere Zonen aufgeteilt sein. Es kann vorgesehen sein, dass der Drahtkorb in mindestens fünf Zonen aufgeteilt sein kann. Es kann vorgesehen sein, dass die Zonen untereinander keinen elektrischen Kontakt haben und/oder voneinander elektrisch isoliert sein können.

Die Heizmuffe kann beweglich sein. Die Heizmuffe kann den Wirkstoffvorläufer zumindest teilweise umschließen. Die Heizmuffe kann Schleifkontakte aufweisen, die dazu eingerichtet sein können, die einzelnen Zonen des Drahtkorbs mit Strom zu versorgen und/oder zu erhitzen. Die Schleifkontakte können dazu eingerichtet sein, in einer Zone aufgenommenen und/oder bereitgestellten Wirkstoffe zu erhitzen. Die Schleifkontakte können dazu eingerichtet sein, Aerosol zu bilden. Die Schleifkontakte können in einem Bereich und/oder einer Zone den Wirkstoffvorläufer erhitzen. Durch Verschieben der Heizmuffe können mit den Schleifkontakten jeweils entsprechende Bereiche erhitzt sein oder werden, z.B. nacheinander.

Der Evaporator kann ein Schiebeelement aufweisen, das außerhalb der Heizkammer angeordnet sein kann. Das Schiebeelement kann mit der Heizmuffe gekoppelt sein, so dass eine Bewegung des Schiebeelements auf die Heizmuffe und/oder umgekehrt übertragbar sein kann. Durch Verschieben des Schiebeelements kann die Heizmuffe beispielsweise verschoben sein oder werden.

Das Schiebeelement kann mit der Heizmuffe elastisch verbunden sein. Die elastische Verbindung kann ein Silikonschlauch aufweisen, und/oder Das Schiebeelement kann mit der Heizmuffe elastisch durch den Silikonschlauch verbunden sein. Die elastische Verbindung kann ein elektrisch leitendes Material sein oder aufweisen. Das Schiebeelement kann mit der Heizmuffe elastisch durch das elektrisch leitende Material verbunden sein.

Das Schiebeelement kann eine Wirkstoffkammer aufweisen, in die Wirkstoff aufnehmbar sein kann. Wirkstoff kann in der Wirkstoffkammer aufgenommen und/oder bereitgestellt sein oder werden. Es kann vorgesehen sein, dass der Silikonschlauch dazu eingerichtet sein kann, Wirkstoff aus der Wirkstoffkammer in die Heizkammer und/oder die Heizmuffe zu transportieren.

Die Wirkstoffkammer kann durch Druck mit Hilfe einer Membran verkleinerbar sein, so dass Wirkstoff in den Schlauch einspeisbar sein kann. Es kann vorgesehen sein, dass durch Zusammendrücken der Wirkstoffkammer und/oder der Membran Wirkstoff in den Silikonschlauch, die Heizmuffe und/oder die Heizkammer einspeisbar sein kann, und/oder in den Silikonschlauch, die Heizmuffe und/oder die Heizkammer eingespeist, transportiert und/oder befördert sein oder werden kann.

Der Evaporator kann eine Umlenkrolle aufweisen, die dazu eingerichtet sein kann, Wirkstoff in die Heizkammer und/oder die Heizmuffe zu transportieren. Die Umlenkrolle kann beispielsweise konvexe Ausbuchtungen aufweisen. Alternativ oder zusätzlich kann die Umlenkrolle als Schlauchpumpe eingerichtet sein. Die Umlenkrolle kann eine Schlauchpumpe sein oder aufweisen.

Die Heizmuffe kann eine Gehäusekammerwand aufweisen, die ein Filtermaterial aufweisen kann. Es kann vorgesehen sein, dass durch das Filmmaterial Luft und/oder in der Heizmuffe gebildetes Aerosol diffundierbar sein kann, und/oder diffundieren kann. Es kann vorgesehen sein, dass durch das Filmmaterial Aerosol, das in dem von der Heizmuffe umschlossenen Bereich des Wirkstoffträgers gebildet sein kann, diffundierbar sein kann, und/oder diffundieren kann.

Die Gehäusekammerwand und/oder das Filtermaterial kann Poren aufweisen, die dazu eingerichtet sein können, ihre Größe temperaturabhängig zu verändern.

Ein oder das Gehäuse der Heizmuffe kann aus einem Material bestehen oder ein Material aufweisen, das Schadstoffe filtern oder binden kann. Das Material kann ein oder mehrere Zeolithe, bevorzugt inaktivierte Zeolithen mit einer Korngröße von 0,1 bis 2 mm, Marmor, Aktivkohle, Ton, Asche und/oder eine Mischung dieser umfassen, oder daraus bestehen.

Zeolithe können z.B. die Fähigkeit aufweisen, an oder mit negativ geladenen Plättchen Oberflächenkationen auszutauschen und zu binden. Ein Zeolith kann auch als "Molekularsieb" bezeichnet sein oder werden, und/oder ein Molekularsieb sein, aufweisen oder entsprechen, da Zeolithe die unterschiedlichsten Kationen filtern können.

Die Heizmuffe kann eine Dichtung aufweisen. Die Dichtung kann eine Dichtlippe sein oder aufweisen. Die Dichtung kann die Heizmuffe abdichten. Die Dichtung kann dazu eingerichtet sein, dass wenn die Heizmuffe bewegt wird, einen Bereich der Heizkammer, zu komprimieren, und/oder zu verkleinern. Die Dichtung kann dazu eingerichtet sein, dass wenn die Heizmuffe bewegt wird, einen zwischen Dichtung und Mundstück angeordneten Bereich der Heizkammer zu komprimieren, und/oder zu verkleinern. Der Bereich, der durch die Dichtung verkleinert und/oder komprimiert sein oder werden kann, kann in Bewegungsrichtung der Heizmuffe hinter der Dichtung angeordnet sein. Der Bereich kann zwischen Mundstück und Dichtung angeordnet sein.

Die Heizmuffe kann eine oder die Dichtung, bevorzugt eine oder die Dichtlippe, wobei die Dichtung dazu eingerichtet sein kann, dass wenn die Heizmuffe bewegt wird, Luft, Wirkstoff und/oder Aerosol zu fördern. In einigen Ausführungsformen kann die Dichtung dazu eingerichtet sein, dass wenn die Heizmuffe bewegt wird, Luft, Wirkstoff und/oder Aerosol durch ein oder das poröse Gehäuse der Heizmuffe zu fördern.

Es kann vorgesehen sein, dass beim Rauchen, Abrauchen und/oder bei oder nach dem bereichsweisen Erzeugen des Aerosols die Heizmuffe in Richtung des Mundstücks wandern kann. Es kann vorgesehen sein, dass die Heizmuffe in der Heizkammer entlang einer Erstreckungsrichtung des Wirkstoffvorläufers bewegt sein oder werden kann.

Die Heizmuffe kann eine Heizwendel aufweisen. Die Heizwendel kann beheizt sein oder werden. Die Heizwendel kann dazu eingerichtet sein, Wärme abzugeben. Die Heizwendel kann dazu eingerichtet sein, den Wirkstoffträger zumindest bereichsweise zu erhitzen und/oder zu erwärmen.

Die Heizwendel kann eine Memorylegierung aufweisen oder aus einer Memorylegierung bestehen. Die Heizwendel und/oder die Memorylegierung kann dazu eingerichtet sein, sich beim Erhitzen auszudehnen und bei Abkühlen zusammenzuziehen.

Die Heizmuffe kann eine vordere Dichtungsscheibe und eine hintere Dichtungsscheibe aufweisen, zwischen denen die Heizwendel angeordnet sein kann und die mit der Heizwendel verbunden sein können. Die vordere und/oder die hintere Dichtungsscheibe kann dazu eingerichtet sein, ihren Durchmesser zu verändern.

Die vordere Dichtungsscheibe kann näher an dem Mundstück angeordnet sein, als die hintere Dichtungsscheibe. Die hintere Dichtungsscheibe kann weiter weg von dem Mundstück angeordnet sein, als die vordere Dichtungsscheibe.

Die vordere Dichtungsscheibe und/oder die hintere Dichtungsscheibe kann jeweils einen ersten Teil und einen zweiten Teil aufweisen. Es kann vorgesehen sein, dass der erste Teil und der zweite Teil im wesentlichen halbkreisförmig und/oder halbringförmig sein können. Der jeweilige erste Teil kann mit dem jeweiligen zweiten Teil über eine Feder verbunden sein, so dass der Durchmesser der jeweiligen Dichtungsscheibe veränderbar sein kann, beispielsweise bei einer Längenänderung der Feder und/oder wenn die Feder sich ausdehnen oder zusammenziehen kann. Es kann vorgesehen sein, dass die Feder ein Memory-Metall umfassen oder aus einem solchen bestehen kann. Das Memory-Metall und/oder die Feder kann temperaturabhängig expandieren oder sich zusammenziehen. Die Länge der Feder und/oder des Memory-Metalls kann von deren Temperatur abhängen.

Die vordere Dichtungsscheibe und/oder die hintere Dichtungsscheibe kann knickbar sein. Die vordere Dichtungsscheibe und/oder die hintere Dichtungsscheibe kann in oder um einen Winkel von bis zu 130° knickbar sein. Der Durchmesser der jeweiligen Dichtungsscheibe kann durch Knicken zwischen einer ungeknickten Position und einer geknickten Position veränderbar sein. Es kann vorgesehen sein, dass die vordere Dichtungsscheibe und die hintere Dichtungsscheibe dazu eingerichtet sein kann, temperaturabhängig knickbar zu sein.

Die vordere Dichtungsscheibe und/oder die hintere Dichtungsscheibe kann mit einer oder der Innenwand der Heizkammer verklemmt sein und/oder diese kontaktiert, wenn die jeweilige Dichtungsscheibe ungeknickt ist. Die jeweilige Dichtungsscheibe kann in der Heizkammer verschiebbar und/oder beweglich sein, wenn die jeweilige Dichtungsscheibe geknickt ist.

Es kann vorgesehen sein, dass bei einem Temperaturanstieg kann der Winkel, unter dem eine oder beide Dichtungsscheiben geknickt sind, sich nahezu vollständig zurückbilden kann Es kann bei einem Temperaturanstieg eine oder beide Dichtungsscheiben nahezu vollständig in eine oder die ungeknickte Position zurückgeführt sein oder werden kann.

In einigen Ausführungsformen kann damit die Heizmuffe bewegt sein oder werden. Das kann dadurch erreicht werden, dass die hintere Scheibe eine ursprüngliche Form ohne Knick hat. Die hintere Scheibe kann sich an oder mit der Innenwand der Heizkammer klemmen, verklemmen und/oder sperren. Die vordere Scheibe kann nun aktiviert werden, z.B. durch Erwärmung, und Ihren Durchmesser ändern, indem sie in die geknickte Position überführt sein oder werden kann, und/oder indem sie um einen oder den vordefinierten Winkel geknickt ist oder wird. Damit kann die vordere Scheibe bewegbar sein, z.B. im Wesentlichen in eine Richtung entlang des Wirkstoffvorläufers.

Nun kann die Heizwendel aktiviert sein oder werden, z.B. durch Erwärmung, und sich im Wesentlichen horizontal und/oder in einer Richtung entlang des Wirkstoffvorläufers ausdehnen. Dabei kann vordere Scheibe in Richtung Mundstück wandern. Nach dem Heizvorgang kann die vordere Scheibe abkühlen und sich an oder mit der Innenwand der Heizkammer verklemmen und/oder sperren. Nun kann die hintere Scheibe aktiviert werden, z.B. durch Erwärmung. Damit kann die hintere Scheibe ihren Durchmesser verkleinern und der abkühlenden und sich zusammenziehenden Heizwendel folgen. Es sind auch andere Möglichkeiten der Aktivierung der Scheiben, und/oder einer oder der Durchmesseränderung der Scheiben, denkbar. Die Aktivierung muss nicht auf eine thermische Aktivierung begrenzt sein. Beispielsweise können eine oder beide der Scheiben dazu eingerichtet sein, mechanisch und/oder durch mechanische Aktivierung ihren jeweiligen Durchmesser zu ändern.

Der Evaporator kann dazu eingerichtet sein, Aerosol in der Heizkammer durch Mischung von Wirkstoff mit Luft zu erzeugen. Die zugemischte und/oder zuzumischende Luft kann Außenluft sein oder umfassen.

Der Evaporator kann dazu eingerichtet sein, Aerosol durch Mischung von Wirkstoff mit Luft in dem Mundstück zu erzeugen. Die zugemischte und/oder zuzumischende Luft kann Außenluft sein oder umfassen.

Das Mundstück kann einen Aromastoff und/oder Duftstoff umfassen. Der Aromastoff und/oder Duftstoff kann eingekapselt und/oder verkapselt sein. Alternativ oder zusätzlich kann das Mundstück ein poröses Material aufweisen, durch das Aromastoff und/oder Duftstoff hindurchtreten kann.

Der Aromastoff und/oder Duftstoff kann in ein Cellulosepolymer eingekapselt sein. Der Aromastoff und/oder Duftstoff kann ein Cellulosepolymer aus Holzzellstoff, eingekapselt sein.

Das Mundstück kann ein Reservoir für den Aromastoff und/oder Duftstoff aufweisen. Das Mundstück kann dazu eingerichtet sein, den Aromastoff und/oder Duftstoff einem oder dem Aerosol, das durch das Mundstück gefördert ist oder wird, beizugeben und/oder beizumischen. Das Mundstück kann dazu eingerichtet sein, den Aromastoff und/oder Duftstoff einer oder der Luft, die durch das Mundstück gefördert ist oder wird, beizugeben und/oder beizumischen.

Das Mundstück kann als Geschmacksträger und/oder Aromaträger fungieren, z.B. dadurch, dass das Mundstück in dem Reservoir flüssige oder feste Geschmackstoffe und/oder Aromastoffe speichern kann, und/oder das Mundstück einen Geschmackstoff und/oder Aromastoff aufweist oder daraus besteht.

Für die Kapselformmasse wird ein Cellulosepolymer aus Holzzellstoff verwendet. Eine Beschichtung mit Geschmackstoffen zusammen mit Geruch, verstärkt das Empfinden der Inhalation erheblich.

Der Evaporator kann eine im Mundstück angeordnete Klappe aufweisen, die dazu eingerichtet sein kann, auf Sog zu reagieren. Es kann vorgesehen sein, dass wenn die Klappe auf einen oder den Sog durch das Mundstück reagiert, ein Steuerelement und/oder die Heizmuffe aktiviert sein oder werden kann. Ein oder das Aktivieren der Heizmuffe kann ein Aufheizen der Heizmuffe und/oder eine Wärmeabgabe der Heizmuffe umfassen. Alternativ oder zusätzlich kann vorgesehen sein, dass wenn die Klappe auf einen oder den Sog durch das Mundstück reagiert, ein oder das Aerosol in der Heizkammer und/oder in dem Mundstück gebildet wird.

Das Mundstück kann eine Düse aufweisen, durch die Aerosol und/oder Luft aus dem Mundstück ausführbar sein kann.

Der Wirkstoffvorläufer kann dazu eingerichtet sein, Wirkstoff aufzunehmen und/oder bereitzustellen. Der Wirkstoffvorläufer kann eine Aufnahme aufweisen. In der Aufnahme kann Wirkstoff aufgenommen und/oder bereitgestellt sein.

Die Aufnahme kann einen oder mehrere Bereiche aufweisen. In der Aufnahme können in mindestens zwei verschiedenen Bereichen des Wirkstoffvorläufers jeweils verschiedene Wirkstoffe aufgenommen sein.

Der Wirkstoff kann in einer Wirkstoffbereitstellungseinheit aufgenommen sein. Die Wirkstoffbereitstellungseinheit kann in der Aufnahme aufgenommen sein. Wirkstoffbereitstellungseinheit kann eine Zigarette sein oder aufweisen, und/oder eine Zigarettenform haben. Die Wirkstoffbereitstellungseinheit kann ein Tank und/oder einen Behälter umfassen. Die Wirkstoffbereitstellungseinheit kann eine Kapsel und/der Verkapselung sein oder aufweisen. Die Wirkstoffbereitstellungseinheit kann kugelförmig sein.

Der Wirkstoffvorläufer kann ein Filtermaterial aufweisen und/oder aus einem Filtermaterial bestehen. Das Filtermaterial kann eingerichtet sein, bei einem Heizvorgang entstehende Schadstoffe zu binden. In einigen Ausführungsformen kann das Filtermaterial Zeolith umfassen, und/oder einen oder mehrere Zeolithe aufweisen oder daraus bestehen.

Der Wirkstoff kann verkapselt sein. Der Wirkstoff kann in Kugeln verkapselt sein. Eine Hülle der jeweiligen Kapseln und/oder Kugeln kann dazu eingerichtet sein, Schadstoff zu binden und/oder zu filtern.

Der Wirkstoff kann fest, flüssig, und/oder gasförmig sein. Es kann vorgesehen sein, dass der Wirkstoffvorläufer dazu eingerichtet sein kann, Wirkstoff in festem, flüssigen und/oder gasförmigem Zustand aufzunehmen.

Der Wirkstoffvorläufer kann dazu eingerichtet sein, Wirkstoffe zu binden, und bei einer weiteren Aktivierung, bevorzugt bei einer Erhitzung, freizugeben. In einigen Ausführungsformen kann der Wirkstoffvorläufer dazu eingerichtet sein, Wirkstoffe, die bei der Decarboxylierung von Cannabis entstehen, zu binden, und bei einer weiteren Aktivierung, bevorzugt bei einer Erhitzung, freizugeben.

Der Evaporator kann dazu eingerichtet sein, Wirkstoffe, insbesondere CBD und/oder THC aus Pflanzenmaterial zu extrahieren. Der Evaporator kann dazu eingerichtet sein, Wirkstoffe, insbesondere CBD und/oder THC aus Pflanzenmaterial zu extrahieren. Der Evaporator kann dazu eingerichtet sein, Wirkstoffe, insbesondere CBD und/oder THC, in einem Medium zu speichern. Das Pflanzenmaterial kann eine Cannabispflanze oder Teile dieser umfassen.

Das Medium und/oder der Wirkstoff kann gemahlene Cannabisblüten, und/oder eine Mischung aus gemahlene Cannabisblüten und Mehl, Wasser, Butter, Backpulver, und/oder Zucker umfassen.

Der Evaporator kann eine Kupplung aufweisen, die dazu eingerichtet sein kann, ein Zusatzgerät am Evaporator anzudocken und/oder mit dem Evaporator zu verbinden. Das Zusatzgerät kann beispielsweise ein Tank, bevorzugt ein Tank zur Speicherung oder Bereitstellung gasförmiger Stoffe, eine Vakuumpumpe, und/oder ein Nassfilter sein oder aufweisen. Das Zusatzgerät kann eine Batterie und/oder einen Akkumulator umfassen, um z.B. elektrische Energie insbesondere für die Heizmuffe und/oder die Heizwendel bereitzustellen.

Der Evaporator kann einen Flüssigkeitsspeicher aufweisen, der an oder im Mundstück angeordnet sein kann. Der Flüssigkeitsspeicher kann als Nassfilter eingerichtet sein. Der Flüssigkeitsspeicher kann derart angeordnet sein, dass erzeugtes Aerosol und/oder Luft durch den Flüssigkeitsspeicher führbar sein kann. In einigen Ausführungsformen kann vorgesehen sein, dass der Flüssigkeitsspeicher derart angeordnet sein kann, dass erzeugtes Aerosol und/oder Luft durch den Flüssigkeitsspeicher geführt werden muss, bevor es den Evaporator verlassen kann.

Der Flüssigkeitsspeicher und/oder der Nassfilter kann dazu eingerichtet sein, erzeugtes Aerosol zu reinigen und/oder mit Geschmack und/oder Geruch zu versetzten. In einigen Ausführungsformen kann der Flüssigkeitsspeicher ein oder das Reservoir für Aromastoffe und/oder Duftstoffe sein oder aufweisen.

Die Heizmuffe kann dazu eingerichtet sein, den Wirkstoffträger auf eine Temperatur von bis zu 300°C, bevorzugt auf bis zu 180°C, aufzuheizen. Die Heizmuffe kann dazu eingerichtet sein, den Wirkstoffträger auf eine Temperatur von 300°C aufzuheizen. Die Heizmuffe kann dazu eingerichtet sein, den Wirkstoffträger auf eine Temperatur von 220°C aufzuheizen. Die Heizmuffe kann dazu eingerichtet sein, den Wirkstoffträger auf eine Temperatur von 180°C aufzuheizen.

Der Evaporator kann dazu eingerichtet sein, eine Zigarette, z.B. eine handelsübliche Zigarette, nach dem Prinzip "Heat not Burn" zu rösten und/oder auf eine Temperatur bis max. 300°C zu erhitzen, ohne dass dabei Schadstoffe durch Verbrennen freigesetzt werden kann. Durch die bewegliche Heizmuffe kann die Zigarette wie bei einem normalen Rauchvorgang von vorne nach hinten in Stufen erhitzt sein oder werden, beispielsweise in Stufen von 3-5 mm.

Die Erfindung wird anhand der Figuren beispielhaft weiter erläutert. Es zeigen:
- Fig. 1:: eine beispielhafte Ausführungsform eines erfindungsgemäßen Evaporators;
- Fig. 2:: eine andere beispielhafte Ausführungsform eines erfindungsgemäßen Evaporators;
- Fig. 3:: Beispielhafte Heizmuffen einiger Ausführungsbeispiele erfindungsgemäßer Evaporatoren;
- Fig. 4:: eine weitere beispielhafte Ausführungsform eines erfindungsgemäßen Evaporators; und
- Fig. 5:: Ausschnitte einiger Ausführungsbeispiele erfindungsgemäßer Evaporatoren.

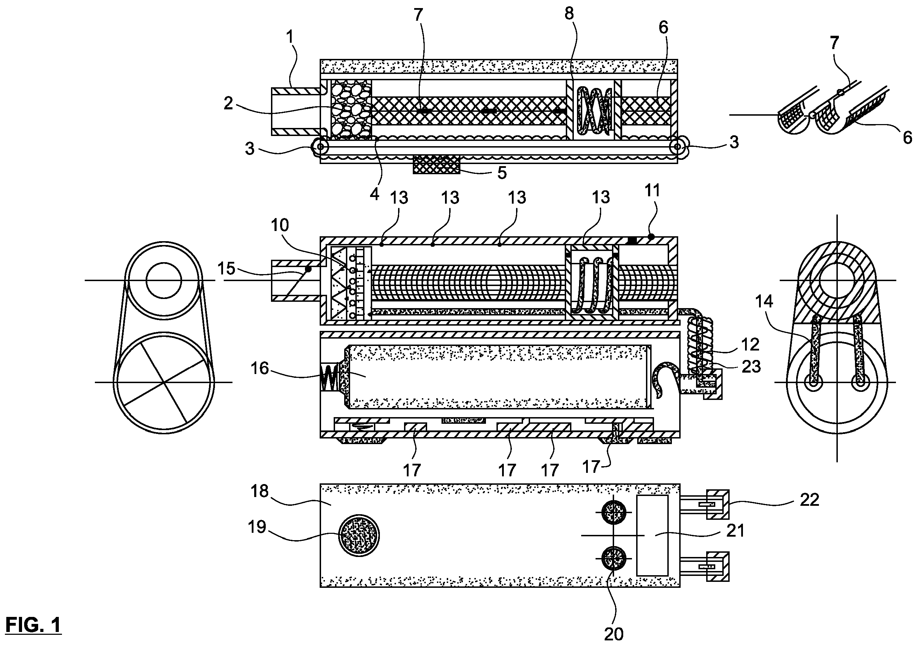

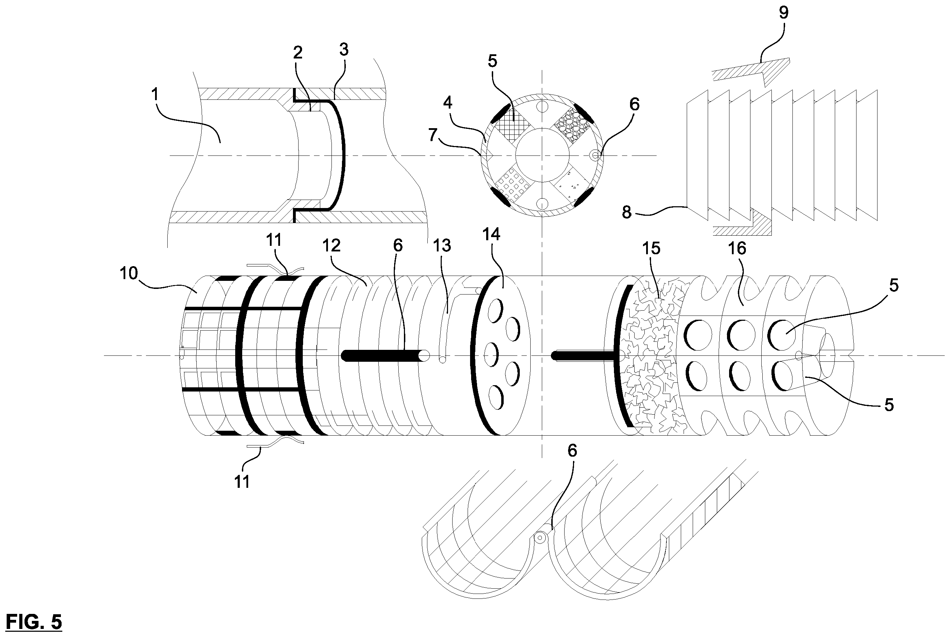

Eines, mehrere oder alle der in einer der Figuren 1, 2, 3, 4 und 5 beispielhaft gezeigten Ausführungsbeispiele erfindungsgemäßer Evaporatoren können mindestens eines, mehrere oder alle Merkmale und/oder Vorteile der in den jeweils anderen Figuren 5 beispielhaft gezeigten Ausführungsbeispiele aufweisen.

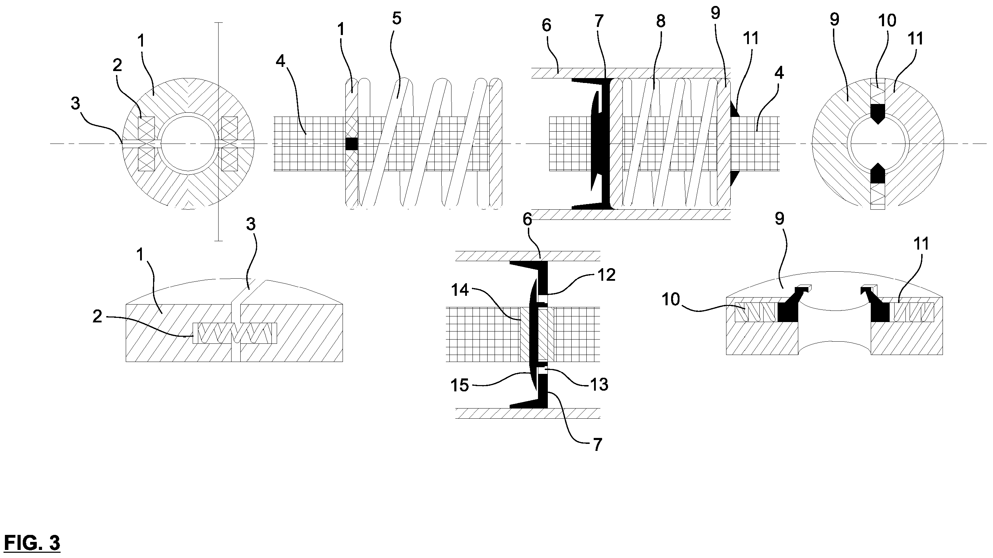

Figuren 1, 2, 4 und 5 zeigen jeweils Ausführungsbeispiele erfindungsgemäßer Evaporatoren. Figur 3 zeigt beispielhafte Heizmuffen einiger Ausführungsbeispiele.

Der Evaporator weist eine Heizkammer und einen Wirkstoffvorläufer auf. Der Wirkstoffvorläufer kann in der Heizkammer angeordnet sein. Der Wirkstoffvorläufer weist die Form eines Hohlzylinders auf, und/oder ist zylinderförmig. Der Evaporator weist eine Heizmuffe auf. Die Heizmuffe umgibt den Wirkstoffvorläufer und ist relativ zu diesem beweglich angeordnet. Die Heizmuffe kann in der Heizkammer angeordnet sein. Der Wirkstoffvorläufer weist eine oder mehrere Bereiche auf. Die Heizmuffe kann einen Bereich überdecken. Wenn die Heizmuffe entlang des Wirkstoffvorläufers verschoben und/oder bewegt ist oder wird, kann die Heizmuffe einen davon verschiedenen anderen Bereich überdecken.

In dem Wirkstoffvorläufer kann ein Wirkstoff aufgenommen und/angeordnet sein. Der Wirkstoff kann z.B. in einer Wirkstoffbereitstellungseinheit aufgenommen und/oder von einer Wirkstoffbereitstellungseinheit bereitgestellt sein oder werden. Die Wirkstoffbereitstellungseinheit kann beispielsweise eine Zigarette sein, die den Wirkstoff enthalten kann. Alternativ oder zusätzlich kann die Wirkstoffbereitstellungseinheit eine Kapselung sein oder aufweisen, in der Wirkstoff aufgenommen und/oder verkapselt sein kann. In einigen Ausführungsformen kann die Wirkstoffbereitstellungseinheit ein Behälter, ein Tank, oder dergleichen, sein oder aufweisen, in dem Wirkstoff aufgenommen und/oder angeordnet sein kann. Die Wirkstoffbereitstellungseinheit kann in dem Wirkstoffvorläufer aufgenommen sein. Beispielsweise kann der Wirkstoffvorläufer eine Aufnahme aufweisen, in der Wirkstoff und/oder die Wirkstoffbereitstellungseinheit aufgenommen und/oder angeordnet sein kann.

In einigen Ausführungsformen kann die Länge des Wirkstoffvorläufers ein Mehrfaches und/oder Vielfaches einer Länge der Heizmuffe sein. Das Mehrfache und/oder Vielfache muss nicht ein ganzzahliges mehrfaches und/oder Vielfaches sein.

Die Heizmuffe kann den Wirkstoffvorläufer, und/oder in dem Wirkstoffvorläufer aufgenommenen Wirkstoff und/oder eine in der Wirkstoffvorläufer aufgenommene Wirkstoffbereitstellungseinheit beheizen und/oder erhitzen. Bei Erhitzung des Wirkstoffvorläufers, des Wirkstoffs und/oder der Wirkstoff Bereitstellungseinheit kann Wirkstoff austreten, verdampfen und/oder extrahiert sein oder werden. Der ausgetretene, verdampfte und/oder extrahiert Wirkstoff kann mit Luftgemisch sein oder werden, um beispielsweise ein wirkstoffhaltiges Aerosol zu bilden.

Die Heizmuffe kann in einigen Ausführungsformen manuell und/oder von einem Benutzer verschoben sein oder werden. Beispielsweise kann dazu ein Schieber und/oder ein Schiebeelement vorgesehen sein, dass mechanisch mit der Heizmuffe gekoppelt sein kann. Durch Verschieben des Schiebers kann die Heizmuffe entsprechend verschoben sein oder werden, insbesondere entlang des Wirkstoffvorläufers. Damit können sukzessive verschiedenen Bereiche des Wirkstoffvorläufers erhitzt, und entsprechend bereichsweise Wirkstoff ausgedrückt austreten, verdampfen und/oder extrahiert sein oder werden und/oder bereichsweise entsprechendes Aerosol gebildet sein oder werden.

In einigen anderen Ausführungsformen kann alternativ oder zusätzlich vorgesehen sein, dass die Heizmuffe beispielsweise über einen Antrieb bewegt sein oder werden kann. In einigen Ausführungsformen kann der Antrieb und/oder die Bewegung der Heizmuffe gesteuert und/oder geregelt sein oder werden. Es kann vorgesehen sein, dass die Bewegung der Heizmuffe von der Art und/oder Menge des jeweiligen Wirkstoffs abhängen kann.

In einigen Ausführungsformen kann alternativ oder zusätzlich vorgesehen sein, dass die Heizmuffe sich selbstständig und/oder automatisch entlang des Wirkstoffvorläufers bewegen kann.

Damit kann in einigen Ausführungsformen der in dem Wirkstoffvorläufer und/oder der Wirkstoffbereitstellungseinheit aufgenommene und/oder bereitgestellter Wirkstoff Wirkstoff in der Heizkammer sukzessive verdampft, extrahiert und/oder geraucht sein oder werden.

Der Evaporator kann ein Mundstück aufweisen. Das Mundstück kann fluidisch mit der Heizkammer verbunden sein oder werden. Gebildetes und/oder erzeugtes Aerosol, insbesondere wirkstoffhaltiges Aerosol, kann durch das Mundstück austreten und/oder einem Benutzer zugeführt sein oder werden. Damit kann das gebildete und/oder erzeugte Aerosol, insbesondere wirkstoffhaltiges Aerosol, von dem Benutzer geraucht sein oder werden.

Es kann vorgesehen sein, dass der Evaporator einen Filter zwischen Mundstück und Heizkammer aufweisen kann, und/oder ein Filter in der Heizkammer angeordnet sein kann. Der Filter kann dazu dienen, Schadstoffe aus dem Aerosol herauszufiltern. Es kann vorgesehen sein, dass das Aerosol durch den Filter hindurchtreten muss, um in das Mundstück einzutreten.

Die Heizmuffe kann eine Heizwendel aufweisen. Die Heizwendel kann eine Heizspirale sein oder einer solchen entsprechen. Die Heizwendel und/oder die Heizmuffe kann dazu eingerichtet sein, elektrische Energie in Wärme umzuwandeln. Beispielsweise kann die Heizmuffe und/oder Heizwendel ein geeignetes Metall und/oder eine geeignete Metalllegierung aufweisen und/oder daraus bestehen. Die Heizmuffe und/oder Heizwendel kann einen elektrischen Widerstand haben, der derart gewählt ist, dass wenn elektrischer Strom durch die Heizmuffe und/oder Heizwendel fließt, die Heizmuffe und/oder Heizwendel erhitzt und/oder aufgewärmt ist oder wird.

Es kann vorgesehen sein, dass sich der Wirkstoffvorläufer durch und/oder in die Heizmuffe erstrecken kann. In einigen Ausführungsformen kann die Heizwendel um den Wirkstoffvorläufer herumgewickelt sein. Es kann vorgesehen sein, dass die Heizwendel derart angeordnet und/oder geformt sein kann, dass sie den Wirkstoffvorläufer nicht kontaktiert. In einigen Ausführungsformen kann die Heizmuffe eine vordere und eine hintere Dichtungsscheibe aufweisen. Eine oder beide Dichtungsscheiben können derart geformt sein, dass sich der Wirkstoffvorläufer durch die jeweilige Dichtungsscheibe erstrecken kann. Es kann vorgesehen sein, dass die Heizwendel mit den Dichtungsscheiben verbunden sein kann, und/oder an den Dichtungsscheiben befestigt sein kann. Es kann vorgesehen sein, dass die Dichtungsscheiben elektrisch von der Heizwendel isoliert sein können.

Der Evaporator kann einen elektrischen Energiespeicher, z.B. eine Batterie und/oder einen Akkumulator aufweisen. Der elektrische Energiespeicher kann dazu eingerichtet sein und/oder dienen, elektrische Energie, beispielsweise Strom, bereitzustellen. Es kann vorgesehen sein, dass der elektrische Energiespeicher die Heizmuffe und/oder die Heizwendel mit elektrischer Energie, insbesondere Strom, versorgen kann.

Weitere Vorteile und/oder Merkmale werden nachfolgend beschrieben.

Ein oder das Kondensat kann in einem dafür vorgesehenen Superabsorber (Fig.1. 10) gesammelt werden, welcher ein Teil einer oder der Filtereinheit sein kann. Die Innenwand der Heizkammer (Fig.1. 7) kann mit einer mikro- und nanoskopischen Architektur der Oberfläche versehen sein, die die Haftung von Schmutzpartikeln minimieren kann. Im unteren Teil der Innenflächen der Heizkammer (Fig.1. 7) können Flächen mit Superabsorber (Fig.1. 10) Streifen angebracht sein. Nach dem Inhalationsvorgang kann die Heizkammer (Fig.1. 7) einmal geöffnet werden und der Superabsorber (Fig.1. 10) entnommen werden.

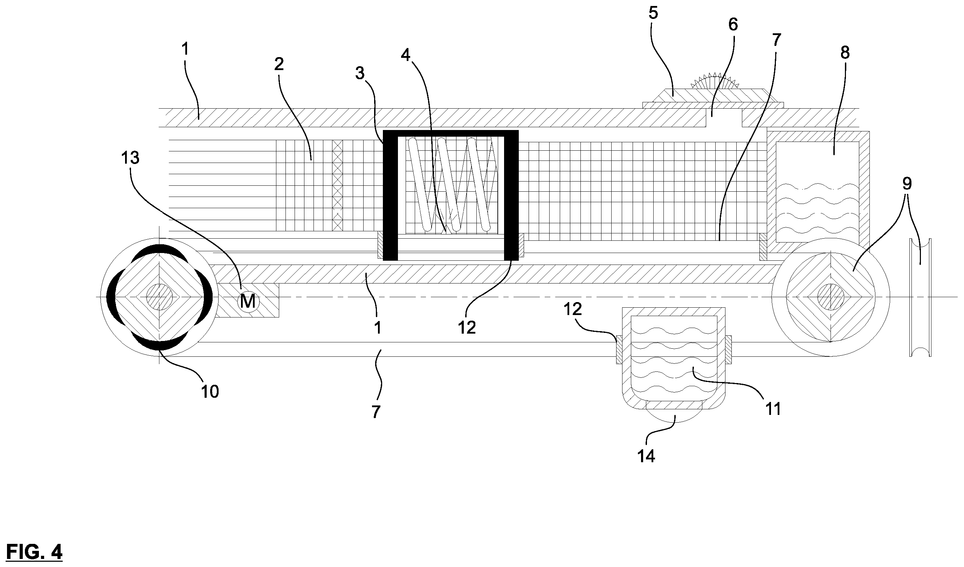

Die Erfindung, und/oder erfindungsgemäße Evaporatoren, können bei jeder Inhalation nur einen geringen Teil an Wirkstoffvorläufers (Fig.3. 4) beheizen. Das wird dadurch erreicht, dass ein hohlzylinderförmiger Wirkstoffvorläufer mit den ungefähren Abmessungen einer Zigarette in einem rohrförmigen Gehäuse (Fig.1. 18), das vom Durchmesser so groß sein kann, dass der Wirkstoffvorläufer (Fig.3 4) der Zentral in dem Rohr angebracht sein kann umlaufend ein Abstand von mindesten 3 mm zur Inneren Begrenzung des Gehäuserohres (Fig.1. 18) haben kann. Der Wirkstoffvorläufer kann auch in Form einer Kugel vorliegen. Die Heizkammer (Fig.1. 7) kann doppelwandig sein. Zwischen der Inneren und der äußeren Wand kann ein Vakuum vorgesehen sein. Eine Ausführungsform kann vorsehen, dass sich in dem Hohlraum auch andere Stoffe befinden können, z.B. Kühlmittel, oder Stoffe, die in die Heizkammer (Fig.1. 7) diffundieren können und/oder die eine Wirkung auf Schadstoffe haben können, die beim Heizvorgang in der Heizkammer (Fig.1. 7) entstehen können. Diese Wirkung auf Schadstoffe kann ähnlich dem Einfluss von AdBlue bei oder auf Dieselabgas sein. Der Wirkstoffvorläufer kann einen Träger haben. Eine Besonderheit der Erfindung kann sein, dass der Träger vom Wirkstoffvorläufer in einer Zone, die 5%- 10% der Länge des Trägers entsprechen kann, umlaufend von einer Heizmuffe (Fig.4. 3) umgeben sein kann. Die Heizmuffe (Fig.4. 3) kann ihre Energie, bzw. Energie, aus zwei Kontaktschienen, beziehen. Die Kontaktschienen können in ganz bestimmten Bereichen Kontaktflächen haben, um das Heizelement und/oder die Heizmuffe, und/oder Funktionselemente, mit Strom zu beliefern. Das Heizelement kann eine Heizeinheit sein oder umfassen. Die Heizmuffe kann das Heizelement und/oder die Heizeinheit sein oder umfassen. Die Heizeinheit kann ein bedeutender Teil der Erfindung sein, weil sie wie beim Abbrennen, z.B. einer Zigarette, bei jedem Atemzug eine Menge, die der Länge der Heizeinheit entsprechen kann, z.B. max. 5 mm, des Wirkstoffvorläufer beheizen kann. Die Heizeinheit kann einen anderen Bereich, z.B. einen oder den vorderen Bereich der Heizkammer (Fig.1. 7) freigeben, Die Heizeinheit und/oder die Heizmuffe kann in Richtung Filtereinheit wandern und/oder beweglich sein Das Beheizen des Wirkstoffvorläufers kann auch direkt hinter dem Filter beginnen. Dabei kann sich der Heizraum bei jedem Atemzug sich vergrößern und aufgestaute Hitze geringer werden, je weiter sich die Heizeinheit vom Filter bewegen kann. Für die Bewegung der Heizeinheit kann in einigen Ausführungsformen die Temperatur der Heizspirale verwendet werden, die beim Aktivieren entstehen kann. Die Heizeinheit kann mindestens einen Kontaktpunkt, eine Verschlussscheibe (Fig.1. 8) vorne, e Heizspirale, eine Verschlussscheibe (Fig.1. 8) hinten, für Stromversorgung besitzen oder aufweisen, die in einigen Ausführungsformen auch auf den Kontaktschienen parallel vorhanden sein oder geführt sein können. Beim Inhalationsvorgang kann Kontakt 3 aktiviert sein oder werden. Memory Federn können sich ausdehnen und den Durchmesser der Verschlussscheibe vergrößern, die sich an der inneren Gehäusewand (Fig.1. 18) festklemmen kann. Gleichzeitig und/oder dabei kann der Kontakt 2 und 1 aktiviert werden, und die Spule kann ca. 200° heiß werden und sich ausdehnen. Die Memory-Federn der Verschlussscheibe kann derart eingerichtet und/oder so programmiert sein, dass sie sich bei Temperaturveränderungen zusammenziehen können. Die Verschlussscheibe 1 kann mit der Heizspirale in eine vorgegebene Richtung wandern, je nach Programmierung oder Einrichtung um 3-5 mm. Sobald der Aspiriervorgang beendet ist und die Heizspirale abkühlt, kann die vordere Verschlussscheibe durch Vergrößerung des Durchmessers blockieren. Die Heizspiral kann sich zusammenziehen und die hintere Verschlussscheibe (Fig.1. 8) mit sich ziehen, die sich beim Abkühlen wieder ausdehnen kann. Das Verschieben der Heizeinheit kann alternativ oder zusätzlich auch mechanisch von außen mit einem Schieber erfolgen.

Vorteilhafterweise kann der Wirkstoffvorläufer bzw. der Wirkstoffträger nach Benutzen und nach Entfernen des Mundstücks mit Hilfe des nicht in Funktion befindlichen Heizelementes ausgeworfen werden. Zudem ermöglicht die Erfindung, dass der Inhalationsvorgang auch für längere Zeit unterbrochen werden kann.

Ausführungsformen der Erfindung umfassen einen Wirkstoffvorläufer, der auch als Wirkstoffvorläuferträger (Fig.5) oder WV-Träger bezeichnet sein kann.

Der WV-Träger kann die Aufgabe haben, Wirkstoffbestandteile Wirkstoffe, und Materialien, die sich günstig auf die Eigenschaften des erzeugten Aerosols auswirken können, miteinander zu verbinden, und/oder zugeführte Mindestenergie so zu nutzen, dass Wirkstoffbestandteile Wirkstoffe, und Materialien durch Wärme und/oder Hitze, die bei bestimmten Temperaturen freigesetzt werden, und durch die Materialeigenschaften des Wirkstoffvorläuferträgers von Schadstoffen befreit werden. Das kann dadurch erreicht werden, dass ein Teil des Wirkstoffvorläuferträgers aus Zeolithen bestehen oder aufweisen kann. In Poren des Wirkstoffvorläuferträgers kann z.B. gemahlenes Blütenmaterial der Cannabispflanze eingearbeitet sein. Es kann vorgesehen sein, dass beim Erhitzen das CBD oder THC durch das Zeolithematerial diffundieren kann oder muss, wobei Schadstoffe diese Barriere nicht durchbrechen können.

Der Wirkstoffvorläuferträger kann eine technisch bedingte Form haben, die in etwa den Ausmaßen einer Zigarette entsprechen kann. Der Wirkstoffvorläuferträger kann aus mehreren Kammern bestehen, die in einigen Ausführungsformen unterschiedliche Wirkstoffe enthalten und/oder miteinander verbunden sein können. Silikontrennscheiben können beispielsweise jedes Teil und/oder die Kammern des Wirkstoffvorläuferträgers gasdicht voneinander trennen. Eine elektrische Leitfähigkeit kann durch isolierende Dichtungen und/oder Silikonscheiben unterbrochen werden. Jede Kammer des Wirkstoffvorläuferträgers kann unabhängig voneinander aktiviert sein oder werden. Mit aktivieren kann ein Erhitzen einer Kammer und/oder eines in eine Kammer aufgenommenen Wirkstoffs, Wirkstoffbestandteils, oder sonstigen Materials gemeint sein. Mit aktivieren kann Mit aktivieren kann ein freisetzen und/oder freigeben eines in eine Kammer aufgenommenen Wirkstoffs gemeint sein. Mit aktivieren kann ein Mit aktivieren kann ein Erzeugen oder Bilden eines Aerosols gemeint sein, z.B. unter Mischung von Wirkstoff mit Luft, beispielsweise freigesetztem und/oder freigegebenem Wirkstoff und Luft. Die verwendeten Materialien können grundsätzlich wieder verwendbar oder biologisch abbaubar sein.

Die Heizmuffe (Fig.4. 3) kann verschiedene poröse Materialien z.B. Zeolithe, geschäumten Metalle oder dergleichen aufweisen oder aus solchen bestehen. Die porösen Materialien können ermöglichen, dass das Material, das sich in dem Wirkstoffträger befindet und beim Erhitzen Aerosole erzeugen kann, beim diffundieren durch die Kammerwand der Heizmuffe von Schadstoffen befreit sein oder werden kann. In einigen Ausführungsformen kann der Wirkstoffvorläuferträger aus Material bestehen oder ein Material aufweisen, das als Molekularsieb (Fig.1. 2) fungieren kann. Es kann vorgesehen sein, dass die Porengröße entsprechend auf die Schadstoffe abgestimmt sein kann. Bei manchen Stoffen können mehrere Schichten der Wirkstoffvorläuferträgerwand notwendig sein, und/oder eine mehrschichtige Wirkstoffvorläuferträgerwand vorgesehen sein, um eine optimale Filterung zu erzielen. Denkbar ist, dass Wirkstoff und/oder Wirkstoffbestandteile in Kammern des Wirkstoffvorläuferträgers eingebracht sein können. Die Kammern und/oder deren Wände können aus einem Material ausgewählt sein, das seine Porengröße je nach Temperatur der Kammer und/oder deren Wände ändern kann. Der Wirkstoffvorläuferträger kann durch seinen Aufbau und zuführen von Energie eine eigene Funktionseinheit bilden, und/oder chemische Vorgänge ausführen.

Die Erfindung betrifft einen Evaporator für die Bildung eines wirkstoffhaltigen Aerosols Dampf-Luft-Gemisches oder/und eines flüssigen Geschmacksträgers aus Kondensationsaerosols durch Verdampfung mittels einer Heizmuffe (Fig.4. 3) und eines Wirkstoffvorläufers.

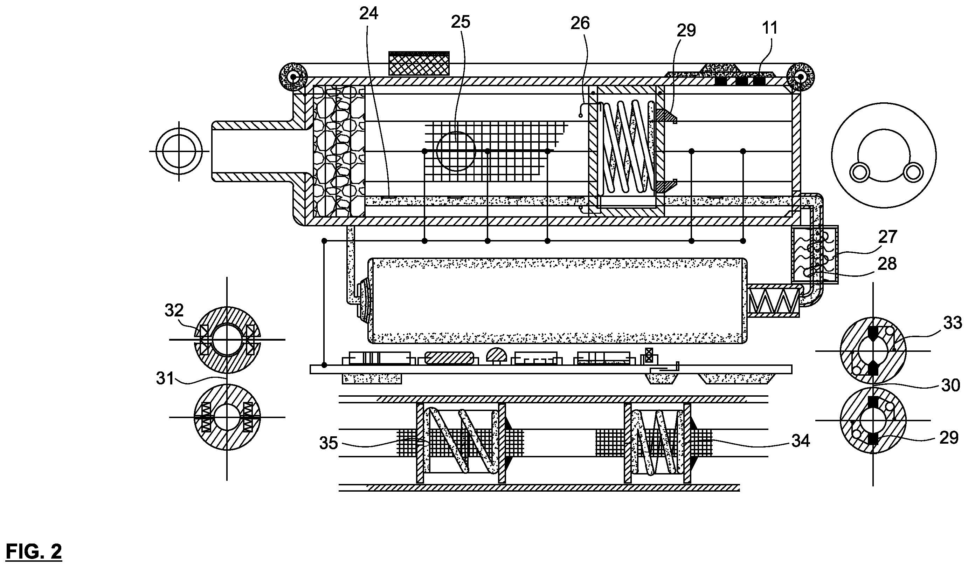

In einigen Ausführungsformen kann der Evaporator ein doppelwandiges Gehäuse (Fig.1. 18) umfassen. In dem Gehäuse (Fig.1. 18) kann ein oder der Wirkstoffvorläuferträger (Fig.1. 6) angeordnet sein. Der Evaporator kann eine Lufteinlassöffnung (Fig2. 11) für die Zufuhr von Luft aus der Umgebung in die Kammer oder Mundstück (Fig.1. 1)) haben. Der Evaporator kann eine oder die Heizmuffe (Fig.4. 3) (Fig. 4. 3) zur Verdampfung einer bestimmten Menge von Wirkstoff in einer oder der Heizkammer.

(Fig. 4. 1) Mit einer oder der in einer oder der Heizkammer angeordneten Heizmuffe (Fig.4. 3) kann ein Aerosol und/oder ein Dampf/ Luftgemisch in der Heizkammer (Fig.1. 7) (Fig.4. 1) erzeugt werden. Das Aerosol und/oder das Dampf/Luftgemisch kann durch Mischung in der Kammer oder im Mundstück (Fig.1. 1) (Fig.1.1) mit der durch die Lufteinlassöffnung (Fig.2.11) zugeführten Luft erzeugt oder gebildet sein oder werden. Es kann sich das wirkstoffhaltiges Dampf-Luft-Gemisch oder/und wirkstoffhaltiges Aerosol bilden. Um gegebenenfalls einen hohen Schadstoffballast im gebildeten Dampf-Luft-Gemisch bzw. Kondensationsaerosol möglichst weitgehend abzuscheiden, kann in einigen Ausführungsformen eine mehrstufige Schadstoffabscheidevorrichtung und/oder ein Filter (Fig.1.10) vorgesehen sein. Die Schadstoffabscheidevorrichtung und/oder der Filter kann eine Zeolithe-Wirkstoffträgereinheit aufweisen (oder aus einer solchen bestehen), z.B. eine mit der Heizkammer (Fig.1. 7) (Fig.1.7) kommunizierende und/oder fluidisch verbundene Zeolithe-Filtereinheit (Fig.1.10). Es kann alternativ oder zusätzlich vorgesehen sein, dass die Schadstoffabscheidevorrichtung und/oder der Filter ein Molekularsieb (Fig.1. 2) ist oder aufweist, das von gebildetem Dampf- Luft-Gemisch oder/und Kondensationsaerosol durchströmbar ist und diese filtern und/oder binden kann.

In der gegenständlichen Patentanmeldung kann sich der Begriff "Evaporator" auf medizinische wie nicht-medizinische Aspirations- und Inhalationsapparate beziehen, und/oder solche umfassen. Es können auch Rauchartikel und Zigarettenersatz-Artikel, wie sie beispielsweise in der Europäische Patentklasse A24F47/00B enthalten sind, gemeint oder umfasst sein, soweit diese dazu bestimmt sind, dem Benutzer ein Dampf-Luft-Gemisch oder/und wirkstoffhaltiges Aerosol, z.B. wirkstoffhaltiges Kondensationsaerosol, darzureichen.

Die Bezeichnung Wirkstoff (Fig.1.6) kann sich auf alle Stoffe, die Wirkstoffe bei der Decarboxylierung freigeben, bei Decarboxylierung ihren Aggregatzustand ändern, und/oder die durch Inhalation verabreicht werden können, beziehen.

Es kann vorgesehen sein, dass der Wirkstoff einen hohen Anteil an Propylenglykol oder/und Wasser aufweisen kann. Damit kann sich eine Verbesserung der Dosierbarkeit des Wirkstoffs, eine allgemeine Verbesserung der Löslichkeit für weitere Inhaltsstoffe in dem Wirkstoff, oder eine Verbesserung verschiedener physikalischer Eigenschaften, wie beispielsweise eine Verringerung der Viskosität des Wirkstoffs, ergeben. Letzterer Effekt kann beispielsweise bei einer Förderung des Wirkstoffs zum Tragen, kommen und/oder wenn der Wirkstoff zerstäubt sein oder werden soll. Wasser kann zudem die Hygroskopizität des gebildeten Aerosols verhindern und so einem Austrocknen der Mundhöhle und des Rachens vorbeugen. Propylenglykol und Wasser können zusammen ferner keimtötend wirken, und dadurch die Hygiene der Aerosolerzeugung und Aerosolverabreichung verbessern.

In eigenen umfangreichen Versuchsreihen zeigte sich unerwartet, dass der Genuss von auf Basis solcher hochverdünnter Wirkstoffe in mit oder von Zug-Inhalations-Geräten und/oder erfindungsgemäßen Evaporatoren erzeugten Kondensationsaerosolen mit nachteiligen organoleptischen Wirkungen, unter anderem mit einem störenden Geschmacksreiz auf der Zungenspitze und den Lippen verbunden sein kann. Die Ursache dafür kann mit dem großen Anteil von Schadstoffen, die beim Verbrennen von biologischem Material wie Cannabis entstehen können, zusammenhängen. Entsprechende Wirkmechanismen sind beispielsweise von dem Rauchen bekannt.

In einigen Ausführungsformen der Erfindung kann der Anteil von Schadstoffen erheblich reduziert werden, da Wirkstoffe wie Tabak, Cannabis, oder Kräutermischungen nur beheizt, nicht aber verbrannt werden können. Durch den Einsatz der neuen Filtertechnologie unter Verwendung von Zeolithen können Schadstoffe gezielt gefiltert / gebunden werden. Trotzdem kann in einigen Ausführungsformen nicht auszuschließen sein, dass beim Erhitzen der biologischen Wirkstoffe schadstoffbelastete Restfeuchte freigesetzt werden kann Das Entstehen von Kondensat kann in einigen Ausführungsformen nicht zu verhindern sein, weil zum Beispiel bei Cannabis üblicherweise eine Restfeuchte von bis zu 20% im Pflanzenmaterial gebunden sein kann. Diese Restfeuchte kann allerdings bei der Decarboxylierung, z.B. beim Erhitzen von Cannabismaterial, nützlich sein. Das Verdampfen der Restfeuchte kann bei einer Temperatur von 100°C beginnen. Während eines ersten Heizvorgangs kann das Aerosol bereits eine Menge von Schadstoffen aufweisen und/oder transportieren. In diesem Fall kann es denkbar sein, dass keine Inhalation gestartet wird. Die erste Heizphase kann im Wesentlichen auch zuständig für die Bewegung der Heizmuffe (Fig.4. 3) sein. Es kann vorgesehen sein, dass bei der Bewegung findet keine Inhalation statt, und/oder nicht inhaliert wird, wenn die Heizmuffe bewegt ist oder wird. Vorteilhaft bei Cannabis ist, dass die erwünschten Wirkstoffe erst ab 150° freigesetzt werden. Bei Zigarettenprodukten können Wirkstoffe, wie z.B. Nikotin, in einigen Ausführungsformen erst bei 250°-300° aktiviert werden.

Der Erfindung kann vorstehend beschriebene und/oder zuvor aufgezeigten Nachteile überwinden. Ausführungsformen der Erfindung kann insbesondere die Aufgabe zugrunde liegen, eine Inhalationsvorrichtung so auszugestalten, dass eine oder mehrere der folgenden Eigenschaften realisiert werden. In einigen Ausführungsformen kann ein Hohlzylinder als Wirkstoffvorläuferträger dienen, (Fig.1.6). Die Wände des Hohlzylinders können Wände offenporig sein und/oder eine Gitterstruktur aufweisen. Der Hohlzylinder kann von einer Heizmuffe (Fig.4. 3) umschlossen sein. Die Heizmuffe kann auf dem Hohlzylinder verschiebbar sein. Die Länge der Heizmuffe (Fig.4. 3) (Fig.4.3) kann in einigen Ausführungsformen ca. 5%-10% der Länge des Hohlzylinders betragen. Die Länge des Hohlzylinders kann etwa 70-100 mm betragen. Bei einer, mehrerer oder jeder Aktivierung der Heizmuffe (Fig.4. 3) kann die Heizmuffe (Fig.4. 3) den Wirkstoffvorläufer und/oder Wirkstoff beheizen. Bei und/oder nach einer, mehrerer oder jeder Aktivierung kann die Heizmuffe entlang des Hohlzylinders bewegt sein oder werden. Beispielsweise kann die Heizmuffe um ca. 3-5 mm auf dem Hohlzylinder wandern, z.B. bei und/oder nach einer, mehrerer oder jeder Aktivierung. Damit kann gewährleistet sein, dass nur ein geringer Teil des Wirkstoffvorläufers beheizt sein oder werden kann. Die Temperatur kann so gewählt sein, dass ein Verbrennen des Wirkstoffs und/oder des Wirkstoffvorläufers ausgeschlossen sein kann. Das Verfahren kann das Abrauchen einer Zigarette simulieren und/oder nachbilden, ohne jedoch Schadstoffe zu erzeugen. Es sind auch andere Materialien benutzbar und/oder verwendbar, die beim Rauchvorgang Wirkstoffe freigeben, insbesondere Cannabis.

Ein Teil der Erfindung kann die Bewegung der Heizmuffe (Fig.4. 3), (Fig.4.3) unter Nutzung von Temperatur betreffen, Die Bewegung kann beispielsweise durch den Einsatz von Memory-Federelementen möglich sein. Die Memory-Federelemente können die Bewegung durch Expandieren oder Komprimieren der Federelemente ermöglichen. Die Heizmuffe kann selbstwandernd sein. Eine oder die Heizmuffe, insbesondere eine oder die selbstwandernde Heizmuffe, (Fig.4. 3) kann vorne und hinten durch verschiebbare Deckelscheiben (Fig.2. 32,33,) verschlossen sein. Die Deckelscheiben können Dichtungsscheiben sein oder aufweisen, oder solchen entsprechen. Die Deckelscheiben können mit der Heizwendel (Fig.3. 8) verbunden sein. Es kann vorgesehen sein, dass die Deckelscheiben nicht mit der Heizmuffe (Fig.4. 3) verbunden sind. Die Deckelscheiben können im Durchmesser getrennt (Fig.2. 33) sein. Die Deckelscheiben können zwei Teile aufweisen. Die Teile können jeweils Halbkreisteile sein. Die zwei Teile können mit einem Nut- und Federmechanismus, und/oder mit einer Feder, verschiebbar verbunden sein. Die zwei halbkreisförmigen Teile (Fig.2. 32) können mit einer Zug- und/oder Druckfeder aus Memorylegierungen verbunden sein. Die Feder und/oder die Federn können so angebracht sein, dass die Deckelscheiben ihren Durchmesser vergrößern oder verkleinern können. Die Bohrung in den Scheiben kann dem Durchmesser des Hohlzylinders. (Fig.1. 6) entsprechen. Der Hohlzylinder kann durch eine oder beide Deckelscheiben hindurchgeführt sein und/oder sich durch eine oder beide Deckelscheiben erstrecken. Der Außendurchmesser der Deckelscheiben kann so gewählt sein, dass bei Aktivierung der Memory-Feder eine Scheibe, insbesondere die hintere Scheibe, sich an der Innenwand der Heizkammer (Fig.1. 7) (Fig.4. 1) oder am Hohlzylinder abstützen kann.

Die vordere Begrenzungsscheibe, bzw. die vordere Deckelscheibe und/oder vordere Dichtungsscheibe, kann in einem Ruhezustand unbeweglich sein und sich an der Innenwand des Gehäuses (Fig.1. 18), und/oder der Innenwand der Heizkammer und/oder des Hohlzylinders, abstützen. Unter Hitze und/oder Wärme, die von der Heizspirale aus Memorylegierung (Fig.3. 5) in der ersten Aufheizphase bei einer Temperatur von ca. 60°-120° ausgehen und/oder abgegeben werden kann, kann sich die Scheibe, bzw. die vordere Begrenzungsscheibe, zusammenziehen. Dabei kann die vordere Begrenzungsscheibe der Heizwendel (Fig.3. 8), die sich bei erreichter Temperatur ausdehnen kann, nachgeben und nach vorne, und/oder in oder entgegen dem Mundstück, wandern. Beim Abklingen der Temperatur kann die Scheibe sich ausdehnen und an ihrer neuen Position festsetzen. Die Heizeinheit kann sich beim Abkühlen zusammenziehen, wobei die hintere Begrenzungsscheibe reagieren und deren Bewegung nach vorne (und/oder in oder entgegen dem Mundstück) folgen kann. Dies kann ermöglicht werden, da sich die Verklemmung (Fig.2. 29) lösen bzw. nur in einer Richtung greifen kann. Es kann vorgesehen sein, dass sich nach dem Abkühlen der vorderen Scheibe die hintere Scheibe erwärmen kann, und/oder dass nach dem Sperren der vorderen Scheibe die hintere Scheibe gelöst werden kann. Es kann vorgesehen sein, dass somit die hintere Scheibe nachrücken und/oder nachziehen, und oder sich bewegen kann.

Alternativ oder zusätzlich kann vorgesehen sein, dass die Heizmuffe (Fig.4. 3) von außen mit einer Schiebevorrichtung (Fig.4. 7) bewegt werden kann. Das kann dadurch erreicht werden, dass die Heizmuffe (Fig.4. 3) mit einem schlauchähnlichen Element verbunden sein kann. Das schlauchähnliche Element, z.B. ein Schlauch und/oder ein Silikonschlauch, kann über mindestens eine Umlenkrolle (Fig.4. 9) verbunden sein. Die Umlenkrolle kann auf den Laufflächen konvexe Ausbuchtungen (Fig.4. 10) haben, um auf den Verbindungsschlauch eine Pumpwirkung auszuüben zu können. Damit kann Material, beispielsweise Wirkstoff, in die Heizmuffe (Fig.4. 3) (Fig.4. 3) transportiert werden. Das Schiebeelement kann einen Hohlkörper/Tank (Fig.4. 12), der eine Wirkstoffkammer sein, aufweisen oder entsprechen kann, aufweisen. Es kann vorgesehen sein, dass in den Hohlraum (Fig.4. 11) bzw. der Wirkstoffkammer Wirkstoff eingebracht und/oder aufgenommen sein oder werden kann. Eine elastische Membran, die z.B. als Druckknopf (Fig4. 14) gestaltet und/oder ausgebildet sein kann, kann einen Transport von Wirkstoff in die Heizkammer (Fig.1. 7) oder Heizmuffe (Fig.4. 3) ermöglichen, insbesondere einen Transport von Wirkstoff aus der Wirkstoffkammer.

In einigen Ausführungsformen kann die zu filternde Menge Aerosol kann stark reduziert sein. Das kann dadurch erreicht sein oder werden, dass das Aerosol, das nicht in der Heizkammer (Fig.1. 7), sondern im Mundstück (Fig.1. 1) mit Frischluft gemischt und/oder erzeugt werden kann. Es kann vorgesehen sein, dass in der Heizkammer und/oder der Heizmuffe Wirkstoff verdampft und/oder zumindest teilweise in einen gasförmigen Zustand überführt sein oder werden kann, der in dem Mundstück mit Frischluft gemischt werden kann.

Es kann dabei vorgesehen sein, dass der Venturi-Effekt genutzt werden kann.

Alternativ oder zusätzlich kann ein oder das Aerosol in der Heizkammer und/oder der Heizmuffe gebildet sein oder werden. Es kann vorgesehen sein, dass Wirkstoff und Frischluft in der Heizkammer und/oder der Heizmuffe gemischt sein oder werden können. Es kann vorgesehen sein, dass in der Heizkammer und/oder der Heizmuffe Wirkstoff verdampft und/oder zumindest teilweise in einen gasförmigen Zustand überführt sein oder werden kann, der in der Heizkammer und/oder der Heizmuffe mit Frischluft gemischt werden kann.

Die Frischluft kann beispielsweise Umgebungsluft und/oder Außenluft sein oder aufweisen. Die Frischluft kann durch eine oder die Lufteintrittsöffnung eingeführt und/oder zugeführt sein oder werden.

Das Mundstück (Fig.1. 1) kann aus einem porösen Material bestehen oder ein solches aufweisen. Es kann vorgesehen sein, dass Aromastoffe und/oder Duftstoffe, z.B. komplexe Düfte, eingekapselt sein können, z.B. in Kunststoffteile einkapselt sein können. Die Aromastoffe und/oder Duftstoffe können über längere Zeiträume hinweg freigesetzt sein oder werden. Für die Kapselformmasse kann ein Cellulose-Polymer, z.B. ein Cellulose-Polymer aus Holzzellstoff, verwendet sein oder werden. Eine Beschichtung mit Geschmackstoffen kann, z.B. zusammen mit Geruch, das Empfinden der Inhalation erheblich verstärken. Das Mundstück (Fig.1. 1) kann Geschmacksstoffe und/oder Geruchsstoffe abgeben. In einigen Ausführungsformen kann das Mundstück (Fig.1. 1) aus einem Material bestehen oder ein Material aufweisen, das Duft speichern und/oder Geschmackstoffe freisetzten kann. Das Mundstück (Fig.1. 1) kann auch als eine auswechselbare Filtereinheit ausgebildet sein, die drehbar und abwinkelbar vorgesehen und/oder ausgebildet sein kann. Die Funktion kann so gestaltet sein wie bei einem knickbaren Trinkhalm. In einigen Ausführungsformen kann der Evaporator Geschmackstoffe aktivieren und/oder freisetzen, die die Zunge und den inneren Nasenraum eines Benutzers mit Aerosolen als Geschmacksträger versorgen können.

Tatsächlich basiert zumeist nur ein kleiner Teil der als Geschmack (in weiterem Sinn) wahrgenommenen Empfindungen auf Reizen der Geschmacksrezeptoren. Der oft bei weitem überwiegende Teil wird häufig durch flüchtige Aromastoffe hervorgerufen, die das Riechepithel im obersten Nasengang reizen können. Als Beispiel kann das Rauchen einer Pfeife dienen, bei dem ein sich neben einem Pfeifenraucher befindlicher Passivrauchen einen sehr angenehmen Duft vernimmt. Diesen Geruch nimmt der Pfeifenraucher allerdings beim Inhalieren von Tabak selbst nicht wahr. Einige Ausführungsformen erfindungsgemäßer Inhalatoren geben können deshalb nicht nur Wirkstoffe ab, sondern können auch die Zunge und/oder das Riech Zentrum der Nase eines Benutzers manipulieren.

So kann in einigen Ausführungsformen vorgesehen sein, dass beim Inhalieren Aromen und flüchtige Bestandteile erzeugten Aerosols über den Rachen und die Choane in die Nasenhöhle eines Benutzers gelangen können, wo sie von den Riechzellen registriert werden können. Um den Rauchgenuss und den Geschmack zu verbessern, kann vorgesehen sein, dass die Nase von außen, oder intraoral mit Aromastoffen versorgt werden kann. Der Geruch kann direkt aus dem erzeugten Wirkstoff resultieren, oder durch spezielle Aromen, die z.B. im Mundstück (Fig.1. 1) und/oder einem Mundstückmaterial eingearbeitet sein können, ausgehen. Zusätzlich kann das Mundstück (Fig.1. 1) aus Trägermaterial bestehen, das bei Feuchte oder Lippenkontakt über den Speichel Geschmackstoffe und Aromastoffe in den Mundinnenraum transportieren kann. Der Speichelgeschmack kann von den Rezeptoren auf der Zunge als echter Geschmack empfunden werden.

Die Steuerung der Inhalationsphase kann in einigen Ausführungsformen eine bevorzugt im Mundstück (Fig.1. 1) angebrachte Klappe übernehmen, durchführen und/oder initiieren. Die Klappe kann auf Sog reagieren. Die Klappe kann eines oder mehrere Steuerelemente aktivieren, z.B. Heizeinheit. Die Klappe kann die Heizmuffe aktivieren. Die Klappe kann so im Mundstück (Fig.1. 1) angeordnet, und/oder so gestaltet, sein, dass ein Teil des Aerosols beim Inhalieren durch Düsen in Richtung Nase aus dem Mundstück (Fig.1. 1) entweichen kann.

Der Evaporator kann ein Display und/oder einen Bildschirm aufweisen. Das Display und/oder der Bildschirm kann an oder auf dem Gehäuse (Fig.1. 18) des Evaporators angeordnet sein. Das Display und/oder der Bildschirm kann ein Touchdisplay sein, und/oder die Funktionen eines Touchscreens besitzen. Damit kann in einigen Ausführungsformen eine separate Handy-App oder dergleichen überflüssig sein. Alternativ kann der Evaporator mit oder von einer Handy-App gesteuert sein oder werden, und/oder in Datenaustausch stehen. Alternativ oder zusätzlich kann der Evaporator einen Schalter aufweisen, der beispielsweise an oder auf dem Gehäuse angeordnet sein kann.

In einigen Ausführungsformen kann ein Schalter und/oder ein Display auf der Oberfläche des Geräts verschiebbar, in einigen Ausführungsformen zueinander verschiebbar, angeordnet sein. Der Schalter und/oder das Display kann ein magnetisches Trägermaterialaufweisen. Das magnetische Trägermaterial kann in einigen Ausführungsformen dazu eingerichtet sein, auf einer glatten Metallfläche zu haften und von dieser gelöst zu werden. In einigen Ausführungsformen kann ein Benutzer somit seine Funktionstasten dort bzw. so platzieren, dass sich für ihn eine bequeme Handhabung ergeben kann. Die Steuerung des Displays kann so programmiert und/oder eingerichtet sein oder werden, dass sie nur auf einer Fläche aktiv ist, auf der der Benutzer den Kontakt mit den aufgesetzten Elementen hat. Die Oberfläche des Gerätes kann elektrisch leitende Bahnen aufweisen oder Induktion nutzen.

Der Wirkstoffvorläuferträger kann aus einem Zeolithrohr bestehen und/oder aufweisen, und/oder der Wirkstoff kann in einer Kugel, welche mit Wirkstoff und/oder Wirkstoffbestandteilen gefüllt sein kann verkapselt sein. Beim Erhitzen des Zeolithrohres und/oder der Kugel, kann Wirkstoff und/oder Aerosole freigesetzt sein oder werden. Der Wirkstoffdampf kann eine Außenwandbarriere passieren, die in diesem Falle als Molekularsieb (Fig.1. 2) fungieren kann, das dazu eingerichtet sein kann nur gewünschte Substanzen durchzulassen. Die Außenwandbarriere kann ein Teil und/oder eine Außenwand des Zeolithrohrs und/oder der Kugel sein. Das Zeolithrohr und/oder die Kugel kann die Außenwandbarriere aufweisen.

Der Wirkstoff kann in einer oder mehreren Kapseln, beispielsweise Kugeln, verkapselt sein. Die Kapseln können trennbar miteinander verbunden sein und/oder unterschiedliche Materialien und/oder Formen aufweisen. Die Materialien des Wirkstoffvorläuferträgers und/oder der Kapseln können Wirkstoffe, die zum Beispiel bei der Decarboxylierung von Cannabis entstehen, binden und/oder bei weiterer Aktivierung freigeben. Damit kann in einigen Ausführungsformen der Evaporator auch dazu genutzt werden, CBD/THC aus der Cannabis-Pflanze als Wirkstoff zu extrahieren und in einem Medium zu speichern. Der gespeicherte Wirkstoff kann bei einem späteren Aktivieren dann wieder freigegeben werden.

Als besonderes Medium kann beispielsweise gemahlenes Cannabisblütenmaterial in einer Mischung aus Mehl, Wasser, Butter, Backpulver, Zucker vorgesehen sein, das in einigen Ausführungsformen alternativ oder zusätzlich gerollt sein kann, z.B. zu in eine #3 Backform gerollt sein kann. Das Medium und/oder die Backform kann den Ausmaßen des Wirkstoffvorläuferträgers entsprechen und/oder bei einer Temperatur von 110° gebacken werden. Dieses Backwerk kann in dem Evaporator z.B. bei Temperaturen von 180°C-200°C fertig gebacken werden. Bei Temperaturen von 180°C bis 200°C können CBD und/oder THC frei werden, so dass diese und/oder z.B. damit gebildete Aerosole inhaliert werden können. Das gebackene Backwerk kann nach dem fertigen Backen verzehrt werden.

In einigen Ausführungsformen kann der Evaporator eine Kupplung aufweisen. Die Kupplung kann dazu eingerichtet sein, ein Zusatzgerät und/oder ein Zusatzsystem anzudocken und/oder mit dem Evaporator zu verbinden. Beispielsweise kann ein Zusatzgerät dazu eingerichtet und/oder vorgesehen sein, um im Evaporator erzeugte Wirkstoffe und/oder erzeugte Aerosole zu speichern. In einigen Ausführungsformen kann ein Zusatzgerät ein Tank, z.B. ein Tank für gasförmige Stoffe zum Inhalieren, ein Tank und/oder eine Vorrichtung zur Sauerstoffbeimischung, ein Tank für Flüssigkeiten, ein Nassfilter oder dergleichen aufweisen oder sein. Das Zusatzgerät kann eine Batterie und/oder einen Akkumulator umfassen, um z.B. elektrische Energie insbesondere für die Heizmuffe und/oder die Heizwendel bereitzustellen.

Die Heizkammer (Fig.1. 7) kann so beschichtet sein, dass eine mikro- und/oder nanoskopische Architektur der Oberfläche eine Haftung von Schmutzpartikeln auf der Oberfläche minimieren kann. Alternativ oder zusätzlich kann einer saugfähigen Fläche vorgesehen sein, die einen Superabsorber aufweisen oder aus einem Superabsorber bestehen kann. In einigen Ausführungsformen kann die saugfähige Fläche an oder bei der Oberfläche der Heizkammer angeordnet sein, und/oder ein Teil der Oberfläche der Heizkammer sein. Die Heizkammer und/oder die Oberfläche der Heizkammer kann eine oder die saugfähige Fläche aufweisen. In einigen Ausführungsformen kann alternativ oder zusätzlich ein Superabsorber vorgesehen sein, der beispielsweise in der Heizkammer angeordnet sein kann. Ein oder der Superabsorber kann ein negativ geladener Superabsorber sein oder aufweisen, Der Superabsorber kann Kieselsäure aufweisen, und/oder aus Kieselsäure bestehen. Der Superabsorber kann Feuchtigkeit aufsaugen. In einigen Ausführungsformen kann die Heizkammer (Fig.1. 7 positiv statisch aufgeladen sein oder werden, sodass Feuchtigkeit auf der Absorberfläche niederschlagen kann. Es kann vorgesehen sein, dass nach dem Inhalationsvorgang die Absorberfläche ersetzt werden kann.

In einigen Ausführungsformen kann der Wirkstoffvorläuferträger aus Keramik bestehen oder Keramik aufweisen, und/oder eine Keramikbackform sein oder aufweisen. In einigen Ausführungsformen kann die Keramikbackform die Geometrie des Mundstücks (Fig.1. 1) haben und/oder aufklappbar gestaltet sein. Es kann vorgesehen sein, dass die Keramikbackform mit einer Mischung aus Cannabismaterial und Zucker befüllt sein kann. Bei einer Temperatur von 185°C kann der Zucker karamellisieren. Eine Temperatur vom 185°C kann ideal sein, um die Wirkstoffe aus der Cannabispflanze zu extrahieren und/oder im Karamell zu speichern. Das Ergebnis, und/oder die Keramikbackform, kann als Mundstück (Fig.1. 1) verwendet werden, welches Geschmack und/oder Wirkstoff abgeben kann.

Ein erfindungsgemäßer Evaporator kann insbesondere für "Heat not Burn" geeignet sein, und/oder. ein "Heat not Burn" Prinzip verwirklichen. Der Evaporator kann dazu eingerichtet sein eine Zigarette, z.B. eine handelsübliche Zigarette, mit einer Temperatur unter 300°C zu rösten, ohne dass dabei Schadstoffe durch Verbrennen freigesetzt werden können. Die Zigarette kann wie bei einem normalen Rauchvorgang von vorne nach hinten in Stufen erhitzt werden, z.B. in Stufen von 3 mm. In einigen Ausführungsformen können bis zu 20 Rauchzügen möglich sein. De Evaporator kann derart eingerichtet sein, dass ein Abbrennen der Zigarette während Rauchpausen ausgeschlossen sein kann, und/oder das Erhitzen kann derart erfolgen, dass ein Abbrennen der Zigarette während Rauchpausen ausgeschlossen sein kann. Es kann vorgesehen sein, dass ein Verhältnis von Wirkstoff zu Luft, z. B: ein Volumenverhältnis von Wirkstoff zu Luft, nicht zu hoch ist. Ein oder das Verhältnis von Wirkstoff zu Luft kann dem einer Zigarette, z.B. einer handelsüblichen Zigarette, entsprechen. In einigen Ausführungsformen kann, je länger der Zug ist, desto mehr Nikotin zu Luft vorliegen und/oder in einem oder dem Aerosol enthalten sein.

Ein zwei-Phasen-Rauchen kann bei Cannabiszigaretten sinnvoll sein. In einer oder der ersten Heizphase kann z.B. Cannabis getrocknet sein oder werden, und/oder in einer oder der zweiten Heizphase ein oder der Wirkstoff freigesetzt sein oder werden. In einigen Ausführungsformen kann eine portionsweise und/oder abschnittsweise Beheizung des Wirkstoffvorläufers durchgeführt sein oder werden. Eine oder die Heizmuffe (Fig.4. 3) kann einen röhrenartigen Wirkstoffträger allseitig umschließen. Bei jedem Inhalationsvorgang kann so viel Wirkstoff erzeugt sein oder werden, dass ein optimales und/oder vorgegebenes Verhältnis zum Inhalationsvolumen, z.B. ein Anteil von Wirkstoff in einem oder dem Inhalationsvolumen oder/oder Aerosol, entstehen kann. Es kann vorgesehen sein, dass sich die Heizeinheit bei jedem Inhalationsvorgang selbständig auf dem Wirkstoffvorläuferträger verschieben kann. Das Verschieben kann einem oder dem Rauchvorgang einer Zigarette entsprechen, und/oder bei oder nach einem Rauchvorgang erfolgen. Der erfindungsgemäße Evaporator kann allerdings derart eingerichtet sein, dass Verbrennung stattfindet. Eine oder die Schadstoffreduzierung bei der Beheizung von Wirkstoffen und/oder Wirkstoffvorläufer, kann dadurch erreicht werden, dass schon bei einer Temperatur der Heizeinheit bzw. der Heizmuffe die zum Beispiel bei Cannabis 150°C bis max. 200°C betragen kann, Wirkstoffe erzeugt werden können. In einigen Ausführungsformen können Wirkstoffe z.B. bereits bei einer Temperatur von 150°C bis max. 200°C extrahiert und/oder wirkstoffhaltiges Aerosol gebildet werden, da die Menge an Wirkstoff, und/oder z.B. die erforderliche zuzuführende Wärme, klein sein kann. Eine Verbrennung kann bei solchen Temperaturen ausgeschlossen sein.

Die Beheizung und/oder Erhitzung kann steuerbar sein. Die Beheizung und/oder Erhitzung kann sehr genau steuerbar sein.

Ein Problem bei bekannten Inhalatoren und Evaporatoren ist die aus der Verbrennung von biologischem Material resultierende Schadstoffbelastung. In einigen Ausführungsformen kann die Schadstoffbelastung allerdings reduziert sein oder werden, und/oder vermieden sein oder werden, da der Wirkstoffvorläufer sich in einer abgeschlossenen Kammer, und/oder der Heizmuffe und/oder der Heizkammer, befinden kann, die von außen beheizt werden kann. In einigen Ausführungsformen kann die Kammer der Heizmuffe entsprechen, und/oder ein Teil der Heizmuffe sein. Durch ein oder das Ausdehnen beim Beheizen und damit verbundenen Volumenänderung kann und/oder muss das flüchtige Material durch die Außenwände des Wirkstoffvorläuferträgers diffundieren. Die Außenwände der Kammer können aus einem Material bestehen oder ein Material aufweisen, das Schadstoffe filtern oder binden kann. Das Material kann eingeschäumtes Metall sein oder aufweisen. Das Material kann alternativ oder zusätzlich die Eigenschaft besitzen, unter Einfluss einer oder der Temperatur seine Porengröße zu verändern. In einigen Ausführungsformen kann das Material eines oder mehrere aus Zeolithe, Calciumoxid, Siliciumoxid, Aluminiumoxid und geringere Mengen an Magnesiumoxid, Manganoxid, Eisenoxid, Phosphorpentoxid und Calciumsulfid sein oder aufweisen. Das Material kann ein Filter sein oder als Filter dienen, und/oder ein Filtermaterial sein. Das Filtermaterial kann beispielsweise eine Vermischung aus Ton, Hochofenschlacke und Kupferoxid sein oder aufweisen.

Der Evaporator kann ein Molekularsieb (Fig.1. 2) aufweisen. Das Molekularsieb kann ein Filter sein oder aufweisen. Der Evaporator kann einen Filter aufweisen, der ein oder das Molekularsieb ist oder aufweist. Ein Molekularsieb kann beispielsweise für natürliche und synthetische Zeolithe oder anderer Stoffe, die eine hohe Adsorptionskapazität für Gase, Dämpfe und gelöste Stoffe mit bestimmten Molekülgrößen haben, umfassen oder sein. Durch eine geeignete Wahl des Molekularsiebs (Fig.1. 2) können Moleküle verschiedener Größen getrennt sein oder werden. Es kann vorteilhafterweise vorgesehen sein, dass nur die Menge Wirkstoff, die in der Kammer ist, das Filterverfahren und/oder den Filter und/oder das Molekularsieb durchläuft. In einigen Ausführungsformen kann vorgesehen sein, nicht das komplette Inhalationsvolumens zu filtern.

Besonders unangenehm beim Inhalieren von Wirkstoffen kann sein, wenn sich Kondensat bildet, das irgendwann in das Mundstück (Fig.1. 1) gelangt. Die Kondensatrückstände können eine Folge der hohen Dampfkonzentration in der Kammer sein, insbesondere in der Nähe einer oder der Verdampfungsfläche bzw. Dampfaustrittsfläche. Die Bildung von Kondensatrückständen kann im Wesentlichen durch eine Diffusion in Richtung kühlerer Begrenzungswände bestimmt sein, und blitzartig erfolgen. In einer Ausführungsform der Erfindung dann vorgesehen sein, das Kondensat zu binden. Das kann dadurch erreicht werden, dass die Innenwände, z.B. der Heizkammer und/oder der Heizmuffe, mit einem Material beschichtet sein kann. Das Material kann beispielsweise einen Lotuseffekt haben und/oder bewirken. Die Heizkammer (Fig.1. 7) kann verschiedene Zonen und/oder Bereiche aufweisen, in denen sich ein Superabsorber (Fig.1. 10) befinden kann. Der oder die Superabsorber kann austauschbar sein.

Eine interessante Beobachtung ist, dass bei allen Inhalatoren und Evaporatoren, und besonders beim Rauchen, häufig ein angenehmer Geschmack und/oder Aroma vermisst wird, oder zumindest wünschenswert wäre. Besonders tritt das bei Rauchern auf. So ist vielerlei Zigarettenherstellern verboten, Geschmackstoffe und Aromastoffe in Ihre Cannabiserzeugnisse beizumischen.

Ein erzeugtes Aerosol kann üblicherweise kaum einen angenehmen Geschmack transportieren oder vermitteln. Geschmack wird üblicherweise auf der Zunge aktiviert. Versuche haben gezeigt, dass bei einer Kombination aus Inhalieren, z.B. über Lunge und/oder intraorale Schleimhaut, riechen, z.B. in Rachen und Nase, und schmecken, z.B. mittels Zunge, ein sehr intensives Geschmackserlebnis beim Inhalieren erzeugt werden kann. Der Geschmack kann z. B: im Wesentlichen über die Nase erfolgen. Erst zusammen mit dem Geruch kann ein Aroma eines Aerosols entstehen und/oder wahrgenommen werden. Es kann häufig einfacher sein, Geruch mit einem oder dem Aerosol zu übertragen, als Geschmack im Mund und/oder auf der Zunge zu erzeugen.

Eine Erzeugung von Geschmack wird häufig nur unter Einbeziehung von Speichel, der die Geschmacksknospen auf der Zunge aktivieren kann, erreicht.

Ausführungsformen der vorliegenden Erfindung können diese Nachteile überwinden. In einigen Ausführungsformen kann vorgesehen sein, Geschmack und/oder Aromastoffe werden ausschließlich über das Mundstück (Fig.1. 1) abzugeben. Dies kann dadurch erreicht werden, dass das Mundstück (Fig.1. 1) teilweise aus Zucker und/oder Abkömmlingen oder Derivate dieser bestehen oder solche aufweisen kann, die mit Geschmack und/oder Geruch, und/oder. Wirkstoffkonzentraten versetzt sein können. Der Geschmack kann über die Lippen aktiviert und gesteuert werden, und in einigen Ausführungsformen dann z.B. auf die Zunge übertragen sein oder werden. Es kann vorgesehen sein, dass auf der Zunge Geschmacksempfindungen provoziert und/oder hervorgerufen werden, die auch gut inhalierbar sein können. Eine weitere Ausführung kann ein entsprechendes Verfahren, z.B. ein vorstehendbeschriebenes Verfahren, umfassen und/oder dazu eingerichtet sein, ein entsprechendes Verfahren durchzuführen. Auch handelsübliche Zigaretten, Zigarettensticks, insbesondere Zigaretten mit Inhaltsstoffen aus der Cannabispflanze, sogenannte Hanfzigaretten, können beheizt werden, ohne zu verbrennen.

Anders formuliert soll in einigen Ausführungsformen ausgehend von einer Inhalationsvorrichtung der eingangs geschilderten Art ein möglichst vollwertiger Zigarettenersatz geschaffen werden, und zwar sowohl hinsichtlich der pharmakologischen und pharmakokinetischen Wirkungen als auch hinsichtlich der organoleptischen Wirkungen des erzeugten Dampf-Luft-Gemisches bzw. Kondensationsaerosols. Die Inhalationsvorrichtung soll in einigen Ausführungsformen ergonomisch zu handhaben sein und möglichst kompakt und raumsparend ausgestaltet werden, so dass eine Verwendung als Taschenevaporator möglich ist.

Ein Evaporator kann ein Inhaliergerät oder eine Inhalationsvorrichtung sein oder aufweisen, und/oder als solche dienen und/oder bezeichnet sein oder werden.

Demnach kann ein Evaporator eine Heizmuffe (Fig.4. 3) aufweisen, die eine Zigarette, bevorzugt auf einer Länge von 3-5 mm, umschließen kann. Wie bei einem Rauchvorgang einer handelsüblichen Zigarette kann bei jedem Zug an der Zigarette die Heizmuffe entlang der Zigarette in z.B. Richtung eines oder des Filters wandert. In einigen Ausführungsformen kann der Evaporator einen oder den Filter aufweisen, der in der Heizkammer und/oder an oder bei dem Mundstück angeordnet sein kann. Es kann ein normales bzw. übliches Abrauchen einer Zigarette simuliert und/oder nachgebildet sein oder werden, aber bei einer von der Heizmuffe (Fig.4. 3) erzeugten Temperatur von max. 300°C. Damit können Wirkstoffe freisetzt sein oder werden, ohne die Zigarette zu verbrennen. Die Zigarette kann sich beispielsweise in einem zylinderförmige Gitterkäfig befinden und/oder angeordnet sein. Der zylinderförmige Gitterkäfig kann welcher sicherstellen, dass die Zigarette nach dem Abrauchen problemlos aus dem Evaporator entnommen werden kann. Es ist auch denkbar, dass der Gitterkäfig aus einzelnen Zonen bestehen oder einzelne Zonen aufweisen kann, die beheizt werden können. In diesem Fall kann die Heizmuffe (Fig.4. 3) als Energieversorger dienen, der eine oder mehrere der jeweiligen Zonen mit Energie versorgen kann.

Ein wesentlicher Teil der im Zuge der Verdampfung zugeführten Wärme kann in der Heizmuffe (Fig.4. 3) verbleiben. Damit kann verhindert sein oder werden, dass die thermische oder innere Energie in der Heizmuffe (Fig.4. 3) den Heizraum, z.B. bei mehrfach hintereinander folgenden Inhalationen, zu sehr aufheizen kann. Mit zunehmender Temperatur kann aber auch der Sättigungsdampfdruck auf die Wände der Heizmuffe (Fig.4. 3) steigen, die als Filtereinheit dienen können, was dazu führen kann, dass der Druck, den das erzeugte Aerosol in der Gasphase auf die Filterelemente und/oder Wände ausübt, relativ hoch sein kann. Dies kann aber nicht schädlich für die Filterfunktion, z.B. der Wände und/oder Filtereinheit, sein.

Die Kondensatrückstände können am Ort ihres Entstehens, z.B. in der Kammer, beispielsweise in der Heizkammer und/oder der Heizmuffe, von einem oder mehreren Superabsorber (Fig.1. 10) aufgenommen, abgeleitet und/oder gespeichert sein oder werden. Es kann vorgesehen sein, dass die Poren des Superabsorbers (Fig.1. 10) in einigen Ausführungsformen weder von der in die Kammer einströmenden Luft noch vom gebildeten Dampf-Luft-Gemisch bzw. Kondensationsaerosol durchströmt sein oder werden können. In einigen Ausführungsformen können die Poren auch nicht dazu dienen, die zu verdampfenden Wirkstoffe zu speichern oder zum Evaporator zu fördern. In einigen Ausführungsformen können die Poren des Superabsorbers (Fig.1. 10) dazu dienen, in mindestens einer Ausführungsform ausschließlich dazu dienen, Kondensatrückstände kapillar zu binden.

Bei einem Luftvolumen, z.B. einem üblichen Luftvolumen, und/oder einem Luftvolumen von 0,5 bis 1,0 L, kann eine kleine Kühlflüssigkeitsmenge, z. B: von 20 bis 80 mL, ausreichen, um eine ausreichende Kühlung zu bewirken. Die Kühlflüssigkeit kann vorzugsweise aus Wasser und/oder anderen Kühlmitteln bestehen oder aufweisen.

Verschiedene Kühlmittel können sich unter anderem durch eine keimtötende und lösungsvermittelnde Wirkung auszeichnen. Zusätzlich können in der Kühlflüssigkeit Konservierungsmittel wie Carbonsäuren, z.B. Sorbinsäure oder Benzoesäure, enthalten sein. Durch die Zugabe von Carbonsäuren kann die Kühlflüssigkeit ferner so weit angesäuert sein oder werden, dass sie in der Lage ist, aus dem Dampf-Luft-Gemisch bzw. Kondensationsaerosol freigesetzte Schadstoff aufzunehmen und/oder zu binden.

Der Evaporator und/oder das Mundstück (Fig. 1. 1) kann eine Flüssigkeitssperre in Form eines Superabsorbers (Fig.1. 10) aufweisen. Der Superabsorber kann z.B. ein Zeolith sein oder aufweisen. Zeolithe stellen für zahlreiche Industrie- und Konsumprodukte wichtige Materialien dar und werden seit einigen Jahrzehnten intensiv genutzt. Zeolithe sind sehr porös, denn ihre gerüstartige Struktur ist von unzähligen nano- bis mikrometergroßen Poren und Kanälen durchsetzt. Sie gleichen in ihrer Struktur einem Schwamm mit vielen (Mikro- oder Nano-) Löchern, sind aber nicht flexibel. Wegen solchen nanoskaligen Poren gehören sie zu den ganz wenigen Materialklassen, die nicht nur deswegen als Nanomaterialien gelten, weil sie nanoskalige Partikel bilden, sondern auch, weil sie Nanoporen besitzen können. Sie sind vor allem als Katalysatoren in chemischen Prozessen von Interesse, da sich Flüssigkeiten frei durch die Poren bewegen können und an deren Wänden chemische Reaktionen ablaufen können. Auch für Filtrationen können sie genutzt werden: wegen der Nanoporen können auch sehr kleine Stoffe getrennt werden, das nennt sich dann Nanofiltration.

Der offenporige Porenkörper bzw. eine oder semipermeable Membran eines oder des Filters und/oder eines oder des Superabsorbers kann für das Dampf-Luft- Gemisch bzw. Wirkstoff durchlässig sein, hydrophoben Materialeigenschaften dieser können jedoch verhindern, dass Schadstoffe in die Kammer gelangen und/oder über das Mundstück (Fig.1. 1) austreten können, in einigen Ausführungsformen beispielsweise unabhängig von der räumlichen Lage und/oder Anordnung in dem Evaporator. Als hydrophobes Material eignet sich grundsätzlich jedes von Natur aus hydrophobem Material sowie durch geeignete Verfahren hydrophobiertes Material. Als typisches Beispiel für ein naturhydrophobes Material sei PTFE (Polytetrafluorethylen) genannt.

Die Erfindung ist jedoch nicht auf die beschriebenen Wirkstoffe und/oder Material, und/oder chemisch inertes Material, beschränkt. In einer bevorzugten Ausgestaltung kann rekonstituierter Cannabis, expandierter Cannabis oder Mischungen derselben vorgesehen sein. Das Cannabis kann beispielsweise als Schnittcannabis oder gemahlener Cannabis vorliegen. Als Aromastoffe eignen sich beispielhaft Cannabisextrakt, Cannabisaromaöle, Menthol, Kaffeeextrakt, Cannabisrauch-Kondensat oder eine flüchtige aromatische Fraktion eines Cannabisrauch-Kondensats. Selbstverständlich ist die Erfindung nicht auf diese Auswahl beschränkt. Die Cannabisfüllung kann aus Schnittcannabis, Feinschnitt-Cannabis, Stopfcannabis, aus einem zigarrenartigen Cannabiswickel oder aus vergleichbaren oder ähnlichen Cannabisformen gebildet werden. Als Cannabis eignen sich insbesondere getrockneter fermentierter Cannabis, rekonstituierter Cannabis, expandierter Cannabis oder Mischungen derselben. Das Cannabis kann zusätzlich gesoßt, gewürzt, aromatisiert oder/und parfümiert werden, indem ihm aromatische Zusätze wie beispielsweise Cannabisextrakt, Cannabisaromaöle, Menthol, Kaffeeextrakt, Cannabisrauch-Kondensat oder eine flüchtige aromatische Fraktion eines Cannabisrauch-Kondensats zugesetzt werden. Meist erzeugen die Wirkstoffvorläufer z.B. Cannabisblütenmaterial beim Erhitzen kein oder wenig Qualm. Das kann durch Beimischung von verschiedenen Stoffen z. B. Propylenglykol im Rahmen des (deutschen) Tabakerzeugnisgesetzes geändert werden.

Zum Transportieren der Wirkstoffe hat sich Propylenglykol bewährt. Der Siedepunkt kann bei ca.188°C liegen, was dem Freisetzen von Wirkstoffen bei Cannabisblüten gleichkommen kann. Der Rauch kann also als sichtbarer Indikator für die Temperatur gedeutet werden.

§ 13 des deutschen Tabakerzeugnisgesetz schreibt vor, dass in Liquid kein Inhaltsstoff (außer Nikotin) enthalten sein darf, der ein Risiko für die menschliche Gesundheit darstellen könnte.

Propylenglykol (PG) E1520 PG (1,2 Propantriol) ist als Lebensmittelzusatzstoff mit der Bezeichnung E1520 zugelassen. PG findet man u.a. auch in Inhalatoren, Nebelanlagen in Diskotheken, Cremes, etc. PG ist eine klare, farblose, nahezu geruchlose und stark hygroskopische Flüssigkeit. Glycerin (VG) E422VG trägt als zugelassener Lebensmittelzusatzstoff die Bezeichnung E 422. VG ist in allen natürlichen Fetten und Ölen als Fettsäureester vorhanden und spielt eine zentrale Rolle als Zwischenprodukt in verschiedenen Stoffwechselprozessen.

Die durch Kondensation erzeugten Wirkstoffaerosolpartikel können in der Regel einen massemedianen aerodynamischen Durchmesser (MMAD) kleiner als 2µm aufweisen. Damit können diese auch die Alveolen erreichen, wo das Wirkstoff in den Blutkreislauf übergehen kann. Dieser Übergang kann schnell und/oder blitzartig erfolgen. Schon etwa 7-10 Sekunden nach der Inhalation kann der Wirkstoff in gebündelter Konzentration sein Zielorgan, nämlich das zentrale Nervensystem, erreichen.

Bei Verwendung von Heizleiterlegierungen anstatt Edelstahl kann ein Heizelementwiderstand, z.B. der Heizmuffe und/oder Heizwendel, deutlich gesteigert werden. Beispielsweise kann sich bei Verwendung von DIN-Werkstoff-Nummer 2.4872 (NiCr20AISi) im Vergleich zu AISI 304/ AISI 316 um den Faktor 1,8 und bei Verwendung von DIN- Werkstoff-Nummer 1.4765 (CrAI255) gar um den Faktor 2,0 ergeben.

Die Temperatur kann in der Heizmuffe (Fig.4. 3) durch einen oder den elektrischen Schaltkreis beliebig gesteuert werden. Bei Versuchen hat sich gezeigt, dass eine Intervallbeheizung das Risiko des Verbrennens des Wirkstoffvorläufers erheblich reduzieren kann. Es kann eine Verwendung von Bimetall in Verwendung mit Memory-Federelementen vorgesehen sein. Bimetallschalter haben sich in vielen Wärmegeräten zum Beispiel bei der Temperaturregelung von Bügeleisen und Boilern, zur Steuerung von Kaffeemaschinen Toastern und Wasserkochern bewährt. In einem oder dem Temperaturschalter kann beispielsweise ein oder der Bimetallstreifen - im Zusammenwirken mit einem Memorymetall oder magnetischem Haften - in Abhängigkeit von der Temperatur einen Kontakt öffnen oder schließen. Der Kontakt kann beispielsweise eine oder die Heizwendel (Fig.3. 8) in der Heizmuffe (Fig.4. 3) einschalten oder ausschalten. Zur Temperatursteuerung des Heizwendel (Fig.3. 8) kann ein Ende des Bimetallstreifens an der Heizwendel (Fig.3. 8) fixiert und das andere Ende mit einem elektrischen Kontakt versehen sein. Die Bimetallstreifen können entweder selbst vom Strom durchflossen sein oder werden, oder eine Heizwicklung tragen.

Wird oder ist beispielsweise ein Bimetallstreifen in eine oder die Zuleitung der Heizwendel (Fig.3. 8) der Heizmuffe (Fig.4. 3) gesetzt und/oder angeordnet, so kann die Heizwendel (Fig.3. 8) nach kurzer Aufheizzeit beginnen zu glühen. Durch das Aufheizen kann der Stromfluss vom Bimetall zur Heizwendel (Fig.3. 8) unterbrochen werden. Beim Abkühlen kann sich der Stromkreis wieder schließen. Entsprechend kann auch das Beheizen der Heizmuffe (Fig.4. 3) erfolgen.

Im Fall von einer metallischen Heizwendel (Fig.3. 8) kann eine einzelne Lithium-Polymer- oder Lithium-Ionen-Zelle mit einem Leerlauf oder Nennspannung von etwa 3,7 V ausreichend sein. Eine einfache Regelungsstrategie kann darin bestehen oder die Schritte umfassen, die Dauer der Energiezufuhr in mehreren Heizzyklen zu unterteilen, beispielsweise in eine Aufheizperiode und eine daran anschließende Verdampfungsperiode. In einem oder dem intermittierenden, zugsynchronen Betrieb des Evaporators kann orientiert sich die Dauer der Energiezufuhr an der Dauer eines Zuges orientieren, und/oder einer solchen entsprechen. In einigen Fällen kann von einer durchschnittlichen Zugdauer von etwa 2,1 sec (+/-0,4sec) ausgegangen werden. Derselbe Wert kann in etwa auch für Zigaretten gelten. Berücksichtigt man, dass auch nach Abschalten der Energiezufuhr, wegen der im Evaporator noch gespeicherten Wärme zu einem gewissen Grad eine Nachverdampfung stattfinden kann, so kann es zweckmäßig erscheinen, die Dauer der Energiezufuhr etwas kürzer zu wählen, z.B. einen Wert im Bereich 1,5 bis 1,8 sec, z.B. für, während oder bei der ersten der zuvor genannten zwei Perioden. Bei der Aufheizperiode kann die Heizmuffe (Fig.4. 3 samt einem Teil der im Wirkstoffträger gespeicherten Wirkstoffe durch das Heizelement aufgeheizt sein oder werden. Es kann vorgesehen sein, dass die Verdampfung der Wirkstoffe erst einsetzen kann, wenn die Temperatur der Heizmuffe (Fig.4. 3) etwa den Siedebereich der niedrig siedenden Fraktionen der Wirkstoffe erreicht hat. Die Aufheizperiode sollte daher möglichst kurz sein. Insofern kann in einigen Ausführungsformen vorgesehen sein, die Akkuspannung in dieser Periode zu schonen und über einen Kondensator mit einem Aussteuerungsgrad oder Duty Cycle von 100% an das Heizelement weiterzugeben. Die Dauer der Aufheizperiode kann von der benötigten Temperatur zur Freisetzung von Wirkstoffen, der Spezifikationen des Evaporators und/oder von der Menge und/oder Zusammensetzung der Wirkstoffe abhängen. Die Aufheizperiode kann < 0,5 sec sein. In der anschließenden zweiten Periode der Verdampfungsperiode kann der Aussteuerungsgrad wesentlich zurückgenommen sein oder werden, und es kann eine oder die eigentliche Verdampfung der Wirkstoffe erfolgen.

Die zugeführte Energie kann in der zweiten Periode primär zur Verdampfung der Wirkstoffe und sekundär zur Deckung von Energieverlusten verwendet sein oder werden. Durch entsprechende Wahl des Aussteuerungsgrades kann die Verdampfungsleistung und damit auch die pro Zug oder Inhalation verdampfte Menge der Wirkstoffe in gewissen Grenzen gesteuert sein oder werden. Eine obere Grenze kann durch Verwendung von Bimetallen zur Temperatursteuerung gesetzt sein. Somit kann ein lokales Austrocknen und Überhitzen des Wirkstoffs ausgeschlossen sein. Durch eine Zurücknahme bzw. Drosselung des Aussteuerungsgrades kann hingegen einer thermischen Zersetzung eines oder des Materials, und/oder des Wirkstoffs, entgegengewirkt sein oder werden.

Die Regelungsstrategie kann beliebig erweitert und verfeinert werden: zum Beispiel kann es sinnvoll sein, auch den Zustand des Akkus in der Regelungsstrategie zu berücksichtigen, da die Akkuspannung mit zunehmender Entladung und zunehmendem Alter des Akkus, vor allem unter Last, deutlich sinken kann. Diesem Effekt kann mit einer Verkürzung des Aussteuerungsgrades begegnet werden. Um diese Korrektur auch in der Aufheizperiode vornehmen zu können, ist es zweckmäßig, die Akkuspannung eines neuen, geladenen Akkus nicht wie früher vorgeschlagen zu 100%, sondern z.B. nur zu 80% auszusteuern, sodass noch genügend Spielraum für eine Anpassung bleiben kann. Zur Schonung der Akkus kann ein Kondensator zum Einsatz kommen. Eine lange Standzeit der Akkus kann notwendig sein, wenn oder weil mit dem Evaporator möglichst viele Inhalationen durchführbar sein sollen.

Enthält oder umfasst der Hohlzylinder beispielsweise 1-2g effektiv nutzbaren Wirkstoff, und/oder stellt solchen bereit, und/oder beinhaltet oder umfasst das Material Wirkstoff in einer Konzentration von typischerweise 1-5 Vol.-%, dann können mit einem erfindungsgemäßen Evaporator bis zu 75 Züge bzw. Inhalationen ausgeführt werden. Beispielsweise können pro Inhalation 100 µg Wirkstoff verdampft sein oder werden. 75 Züge können dabei etwa 8 Zigaretten entsprechen. Werden pro Zug nur 50 µg Wirkstoff verdampft, dann kann t sich die Reichweite auf 150 Züge bzw. Inhalationen erhöhen, welcher Wert etwa 1 Packung Zigaretten entsprechen kann.

Obwohl der Evaporator in den Ausführungsbeispielen flächig ausgeführt sein kann, ist die Erfindung selbstverständlich nicht auf diese Ausführungsformen beschränkt. Der Evaporator kann vielmehr jede beliebige Form aufweisen, solange er eine in der Heizkammer (Fig.1. 7) angeordneten zylinderförmigen Wirkstoffvorläufer bzw. Wirkstoffvorläuferträger und eine. Heizmuffe (Fig.4. 3) aufweist. Die Heizmuffe kann Dampf Austrittsflächen aus Zeolithe aufweisen, aus welchen erzeugter Dampf in die Heizkammer (Fig.1. 7) übertreten und/oder eintreten kann. Schließlich könnte der Evaporator anstatt mit Ohm'scher Wärme auch mit Induktionswärme, Strahlungswärme oder Mikrowellen aufgeheizt werden. Auch nicht-elektrische Wärmequellen können für die Verdampfung der Wirkstoff genutzt werden. Die unmittelbare Nutzung von chemischer Reaktionswärme sei als Beispiel genannt. Abschließend sei noch erwähnt, dass durch die zusätzliche Beimischung von Alkohol der Transport der Wirkstoffe erheblich verbessert werden kann.

### Bezugszeichenliste

**Fig. 1****:**
   1, Mundstück (Fig.1. 1)
   2, Filterelement, Zeolithe, Molekularsieb (Fig.1. 2), 3, Umlenkrolle, Schlauchpumpe für Wirkstoff
   4, Transport Leitung
   5, Schiebeelement, Hohlkörper, Inhalt Wirkstoff
   6, Wirkstoffvorläufer Träger Abkürzung, WV Träger
   7, Wirkstoffkammer,
   8, Verschluss Scheibe (Fig.1. 8)
   10, Superabsorber (Fig.1. 10),
   11, Lufteinlass,
   12, Kontaktschiene
   13, Sensoren,
   14, Energie Leitungen
   15, Verschluss Klappe
   16, Akku
   17, Elektronik
   18, Gehäuse (Fig.1. 1),
   20, Schaltelemente
   21, Display,
   22, Kontakt
   23, Tank
**Fig. 2****:**
   24, Austrittöffnung für Wirkstoffe
   25, Wirkstoffträger Gitterkorb (Fig.4. 2), 26, Temperatur Sensor
   27, Zusatztank
   28, Wirkstoff
   29, Klemmelement
   30, Verschluss Scheibe (Fig.1. 8) H
   31, Verschluss Scheibe (Fig.1. 8) V
   32, Memory Feder aktiv
   33. Memory Feder aktiv
   34, Memory Feder inaktiv
   35, Memory Feder aktiv
**Fig.3****:**
   1, Trennscheibe vorne
   2, Memory Feder
   3, Spalt nach Aktivierung MF (2)
   4, Wirkstoff- Vorläufer Korb,
   5, Memory Feder aktiv
   6, Außenwand Gehäuse (Fig.1. 18)
   7, Dichtungselement
   8, Memory Heizwendel (Fig.3. 8)
   9, Trennscheibe hinten
   10, Memory Feder
   11, Verschluss
   12, Luftdurchlass
   13, Luftdurchlass
   14, Manschette
   15, Dichtlippe
**Fig. 4****:**
   1, Gehäuse (Fig.1. 18)
   2, Wirkstoffvorläufer Träger
   3, Heizmuffe (Fig.4. 3)
   4, Bimetall-Schalter
   5, Schieber für
   6. Lufteinlass,
   7. Silikon Schlauch,
   8, Zusatztank
   9, Umlenkrolle
   10, Schlauchpumpe
   11, Schieber des
   12, Dichtringe,
   13, Stellmotor,
   14 Pump Knopf
**Fig.5****:**
   1, Kapsel im Wirkstoffvorläufer
   2, Verbindung Element
   3, Dichtung,
   4, Zeolithe Körper
   5, Reservoir
   6, Scharnier
   7, Verschluss
   8. Raster
   9, Hacken
   10, Schleifkontakte
   12, Lamellen als Kühlelemente 13, Scharnier
   14, Trennscheibe
   15, Keramik/ Zeolithe Material 16, der Wirkstoffvorläufer

## Patentansprüche

1. Evaporator zum Extrahieren und Filtern von Wirkstoffen aus biologischen Materialien durch Erhitzen, wobei in einer Heizkammer (Fig.1.7) ein Wirkstoffvorläufer (Fig.1. 6) angeordnet ist, wobei der Wirkstoffvorläufer mit einer Heizmuffe (Fig.4. 3) umgeben ist, die verschiebbar auf dem Wirkstoffvorläufer (Fig.1. 6) sitzt, wobei die Länge des Wirkstoffvorläufers ein Mehrfaches der Breite der Heizmuffe (Fig.4. 3) beträgt, wobei bevorzugt die Breite der Heizmuffe ca. 5% bis 25 % der Länge des Wirkstoffvorläufers beträgt, so dass eine Mehrzahl, vorzugsweise ca. 4 bis 15, Teilbereiche des Wirkstoffvorläufers (Fig.4. 2) einzeln erhitzbar sind, so dass von dem Wirkstoffvorläufer bereitgestellter Wirkstoff zumindest bereichsweise rauchbar ist und/oder ein Aerosol, das bereichsweise den von dem Wirkstoffvorläufer bereitgestellten Wirkstoff umfasst, erzeugbar ist, **dadurch gekennzeichnet, dass** der Wirkstoffvorläufer in Form eines Hohlzylinders ausgebildet ist, dessen Außenwände durchlässig für Aerosole sind.

2. Evaporator nach Anspruch 1, der ein Mundstück aufweist, das dazu eingerichtet ist, ein Aerosol und/oder Luft aus dem Evaporator auszuführen und/oder abzugeben.

3. Evaporator nach einem der vorangegangenen Ansprüche, bei dem die Heizkammer (Fig.1. 7) (Fig.4. 1) doppelwandig mit einer Innenwand und einer Außenwand ist, wobei zwischen Innenwand und Außenwand ein Hohlraum gebildet ist, wobei bevorzugt die Heizkammer ein doppelwandiger Zylinder ist, dessen Innendurchmesser bevorzugt mindestens das doppelte eines Durchmessers des Wirkstoffvorläufers (Fig.1. 1) beträgt.

4. Evaporator nach Anspruch 3, bei dem in dem von der Innenwand und der Außenwand der Heizkammer gebildeten Hohlraum ein Vakuum existiert oder sich in dem Hohlraum ein Wirkstoff und/oder Kühlmittel befindet.

5. Evaporator nach einem der vorangegangenen Ansprüche, bei dem eine oder die Innenwand der Heizkammer (Fig.1. 7) (Fig.1. 7) eine Nanobeschichtung aufweist.

6. Evaporator nach einem der Ansprüche 2 bis 5, der an einem dem Mundstück zugewandten Bereich der Heizkammer (Fig.1. 7) einen Filter aufweist, wobei der Filter bevorzugt eines oder mehrere aus Aktivkohle, Schwamm und/oder Nassfilter, statisch aufgeladene Metallwolle, Meltblown-Vlies oder Kombinationen dieser umfasst.

7. Evaporator nach einem der Ansprüche 2 bis 6, der an einem dem Mundstück zugewandten Bereich einen Superabsorber (Fig.1. 10) aufweist, der dazu eingerichtet ist, Kondensat zu sammeln, und/oder bei dem, bei Bezug auf Anspruch 6, der Filter einen oder den Superabsorber umfasst.

8. Evaporator nach einem der vorangegangenen Ansprüche, der eine Leitung aufweist, die dazu eingerichtet ist, Außenluft von einer Lufteintrittsöffnung (Fig.1. 11) des Evaporators in das Mundstück (Fig.1. 1) des Evaporators und/oder in die Heizkammer zu leiten, wobei die Leitung bevorzugt in der Heizkammer und/oder bevorzugt in einem oder dem von der Innenwand und der Außenwand der Heizkammer gebildeten Hohlraum angeordnet ist.

9. Evaporator nach einem der vorangegangenen Ansprüche, bei dem eine Außenwand des Evaporators mit thermo-chromatischem Lack beschichtet ist, der dazu eingerichtet ist, temperaturabhängig seine Farbe zu ändern.

10. Evaporator nach einem der vorangegangenen Ansprüche, **dadurch gekennzeichnet, dass** der Evaporator ein Gehäuse (Fig.1. 18) aufweist, das aus einem magnetischen Drahtgitter besteht oder ein magnetisches Drahtgitter aufweist, wobei das magnetische Drahtgitter flächig mit Kunststoff beschichtet ist, der, wenn Strom durch Drähte des Drahtgitters fließt, die stromleitende Drähte gegeneinander isoliert.

11. Evaporator nach Anspruch 10, der mindestens ein an oder bei einer Oberfläche des Gehäuses angeordnetes Schaltelement aufweist, wobei bevorzugt das Schaltelement induktiv mit mindestens einem Draht und/oder dem magnetischen Drahtgitter gekoppelt ist.

12. Evaporator nach einem der vorangegangenen Ansprüche, bei dem der Wirkstoffvorläufer (Fig.1. 6) Aufnahme, bevorzugt eine zylinderförmige Aufnahme, bildet und/oder derart dimensioniert ist, dass er die mindestens eine Länge von 8 cm, bevorzugt mindestens 8,4 cm, und einen Durchmesser von mindestens 0,6 cm, bevorzugt mindestens 0,8 cm, aufweist, wobei bevorzugt der Wirkstoffvorläufer derart dimensioniert ist, um eine Zigarette komplett aufzunehmen.

13. Evaporator nach einem der vorangegangenen Ansprüche, bei dem der Wirkstoffvorläufer und/oder die Aufnahme einen zylinderförmigen Drahtkorb aufweist, der bevorzugt in mindestens fünf Zonen aufgeteilt ist, wobei bevorzugt die Zonen untereinander keinen elektrischen Kontakt haben und/oder voneinander elektrisch isoliert sind.

14. Evaporator nach Anspruch 13, **dadurch gekennzeichnet, dass** die Heizmuffe (Fig.4. 3) beweglich ist und den Wirkstoffvorläufer (Fig.4. 2) zumindest teilweise umschließt, und wobei die Heizmuffe Schleifkontakte (Fig.5. 11) aufweist, die dazu eingerichtet sind, die einzelnen Zonen des Drahtkorbs mit Strom zu versorgen und/oder zu erhitzen, bevorzugt um in einer Zone aufgenommene Wirkstoffe zu erhitzen und/oder um bevorzugt Aerosol zu bilden.

15. Evaporator nach einem der vorangegangenen Ansprüche, der ein Schiebeelement aufweist, das außerhalb der Heizkammer (Fig.1. 7) (Fig.4. 1) angeordnet ist und das mit der Heizmuffe gekoppelt ist, so dass eine Bewegung des Schiebeelements auf die Heizmuffe und/oder umgekehrt übertragbar ist.

16. Evaporator nach Anspruch 15, bei dem das Schiebeelement (Fig.4. 12) mit der Heizmuffe (Fig.4. 3) elastisch verbunden ist, wobei bevorzugt die elastische Verbindung ein Silikonschlauch (Fig.4. 7) und/oder elektrisch leitendes Material ist oder aufweist.

17. Evaporator nach Anspruch 15 oder 16, bei dem das Schiebeelement eine Wirkstoffkammer aufweist, in die Wirkstoff aufnehmbar ist, und bevorzugt der Silikonschlauch dazu eingerichtet ist, Wirkstoff aus der Wirkstoffkammer in die Heizkammer (Fig.1. 7) und/oder die Heizmuffe zu transportieren.

18. Evaporator nach Anspruch 17, bei dem die Wirkstoffkammer durch Druck mit Hilfe einer Membran (Fig.4. 14) verkleinerbar ist, so dass Wirkstoff in den Silikonschlauch, die Heizmuffe und/oder die Heizkammer einspeisbar ist.

19. Evaporator nach Anspruch 17 oder 18, bei dem der Evaporator eine Umlenkrolle aufweist, die dazu eingerichtet ist, Wirkstoff in die Heizkammer (Fig.1. 7) und/oder die Heizmuffe zu transportieren, wobei bevorzugt die Umlenkrolle (Fig.4. 10) konvexe Ausbuchtungen aufweist und/oder bevorzugt als Schlauchpumpe eingerichtet ist.

20. Evaporator nach einem der vorangegangenen Ansprüche, bei dem die Heizmuffe (Fig.4. 3) eine Gehäusekammerwand aufweist, die ein Filtermaterial aufweist, durch das Luft und/oder in der Heizmuffe gebildetes Aerosol, und/oder in dem von der Heizmuffe umschlossenen Bereich des Wirkstoffträgers gebildetes Aerosol, diffundierbar ist.

21. Evaporator nach Anspruch 20, **dadurch gekennzeichnet, dass** die Gehäusekammerwand und/oder das Filtermaterial Poren aufweist, die dazu eingerichtet sind, ihre Größe temperaturabhängig zu verändern.

22. Evaporator nach einem der vorangegangenen Ansprüche, **dadurch gekennzeichnet, dass** ein oder das Gehäuse (Fig.1. 18) der Heizmuffe (Fig.4. 3) aus einem Material besteht oder ein Material aufweist, das Schadstoffe filtert oder bindet, wobei bevorzugt das Material ein oder mehrere Zeolithe, bevorzugt inaktivierte Zeolithen mit einer Korngröße von 0,1 bis 2 mm, Marmor, Aktivkohle, Ton, Asche und/oder eine Mischung dieser umfasst.

23. Evaporator nach einem der vorangegangenen Ansprüche, bei dem die Heizmuffe (Fig.4. 3) eine Dichtung, bevorzugt eine Dichtlippe, aufweist, die die Heizmuffe abdichtet und/oder die dazu eingerichtet ist, dass wenn die Heizmuffe bewegt wird, einen Bereich der Heizkammer, bevorzugt einen zwischen Dichtung und Mundstück angeordneten Bereich der Heizkammer zu komprimieren, und/oder zu verkleinern.

24. Evaporator nach einem der vorangegangenen Ansprüche, bei dem die Heizmuffe (Fig.4. 3) eine oder die Dichtung, bevorzugt eine oder die Dichtlippe, aufweist, die dazu eingerichtet ist, dass wenn die Heizmuffe bewegt wird, Luft, Wirkstoff und/oder Aerosol zu fördern, bevorzugt durch ein oder das poröse Gehäuse der Heizmuffe zu fördern.

25. Evaporator nach einem der vorangegangenen Ansprüche, wobei die Heizmuffe eine Heizwendel aufweist.

26. Evaporator nach Anspruch 25, bei dem die Heizwendel eine Memorylegierung aufweist oder aus einer Memorylegierung besteht, wobei die Heizwendel und/oder die Memorylegierung dazu eingerichtet ist, sich beim Erhitzen auszudehnen und bei Abkühlen zusammenzuziehen.

27. Evaporator nach Anspruch 25 oder 26, wobei die Heizmuffe eine vordere Dichtungsscheibe und eine hintere Dichtungsscheibe aufweist, zwischen denen die Heizwendel angeordnet ist und die mit der Heizwendel verbunden sind, wobei die vordere und/oder die hintere Dichtungsscheibe dazu eingerichtet ist, ihren Durchmesser zu verändern.

28. Evaporator nach Anspruch 27, bei dem die vordere Dichtungsscheibe und/oder die hintere Dichtungsscheibe jeweils einen ersten Teil und einen zweiten Teil aufweisen, wobei bevorzugt der erste Teil und der zweite Teil im wesentlichen halbkreisförmig und/oder halbringförmig sind, wobei der jeweilige erste Teil mit dem jeweiligen zweiten Teil über eine Feder verbunden ist, so dass der Durchmesser der jeweiligen Dichtungsscheibe veränderbar ist, wobei bevorzugt die Feder ein Memory-Metall umfasst oder aus einem solchen besteht, so dass bevorzugt die Feder temperaturabhängig expandiert oder sich zusammenzieht.

29. Evaporator nach Anspruch 27 oder 28, bei dem die vordere Dichtungsscheibe und/oder die hintere Dichtungsscheibe knickbar ist, bevorzugt in und/oder um einen Winkel von bis zu 130° knickbar ist, so dass der Durchmesser der jeweiligen Dichtungsscheibe durch Knicken zwischen einer ungeknickten Position und einer geknickten Position veränderbar ist, wobei bevorzugt die vordere Dichtungsscheibe und die hintere Dichtungsscheibe dazu eingerichtet ist, temperaturabhängig knickbar zu sein.

30. Evaporator nach Anspruch 29, bei der die vordere Dichtungsscheibe und/oder die hintere Dichtungsscheibe mit einer oder der Innenwand der Heizkammer verklemmt ist und/oder diese kontaktiert, wenn die jeweilige Dichtungsscheibe ungeknickt ist, und die jeweilige Dichtungsscheibe in der Heizkammer verschiebbar und/oder beweglich ist, wenn die jeweilige Dichtungsscheibe geknickt ist.

31. Evaporator nach einem der vorangegangenen Ansprüche, der dazu eingerichtet ist, Aerosol in der Heizkammer (Fig.1. 7) durch Mischung von Wirkstoff mit Luft, bevorzugt Außenluft, zu erzeugen.

32. Evaporator nach einem der Ansprüche 2 bis 31, der dazu eingerichtet ist, Aerosol durch Mischung von Wirkstoff mit Luft, bevorzugt Außenluft, in dem Mundstück (Fig.1. 1) (Fig.1. 1) zu erzeugen.

33. Evaporator nach einem der Ansprüche 2 bis 32, bei dem das Mundstück einen Aromastoff und/oder Duftstoff umfasst, wobei bevorzugt der Aromastoff und/oder Duftstoff eingekapselt ist und/oder bevorzugt das Mundstück ein poröses Material aufweist, durch das Aromastoff und/oder Duftstoff hindurchtreten kann.

34. Evaporator nach Anspruch 33, bei dem der Aromastoff und/oder Duftstoff in ein Cellulosepolymer, bevorzugt ein Cellulosepolymer aus Holzzellstoff, eingekapselt ist.

35. Evaporator nach Anspruch 33 oder 34, bei dem das Mundstück ein Reservoir für den Aromastoff und/oder Duftstoff aufweist und/oder bei dem das Mundstück dazu eingerichtet ist, den Aromastoff und/oder Duftstoff durch das Mundstück gefördertes Aerosol und/oder Luft beizugeben und/oder beizumischen.

36. Evaporator nach einem der Ansprüche 2 bis 35, der eine im Mundstück (Fig.1. 1) angeordnete Klappe (Fig.1. 15) aufweist, die dazu eingerichtet ist, auf Sog zu reagieren, so dass wenn die Klappe auf einen Sog durch das Mundstück reagiert, ein Steuerelement (Fig.1 17) und/oder die Heizmuffe aktiviert wird und/oder ein oder das Aerosol in der Heizkammer und/oder in dem Mundstück gebildet wird.

37. Evaporator nach einem der Ansprüche 2 bis 36, bei dem das Mundstück (Fig.1. 1) eine Düse aufweist, durch die Aerosol und/oder Luft aus dem Mundstück ausführbar ist.

38. Evaporator nach einem der vorangegangenen Ansprüche, bei dem der Wirkstoffvorläufer (Fig.1. 6) dazu eingerichtet ist, Wirkstoff aufzunehmen, und/oder bei dem der Wirkstoffvorläufer eine Aufnahme und in der Aufnahme aufgenommenen Wirkstoff aufweist.

39. Evaporator nach Anspruch 38, bei dem in der Aufnahme in mindestens zwei verschiedenen Bereichen des Wirkstoffvorläufers jeweils verschiedene Wirkstoffe aufgenommen sind.

40. Evaporator nach einem der vorangegangenen Ansprüche, bei dem der Wirkstoffvorläufer ein Filtermaterial aufweist und/oder aus einem Filtermaterial besteht, das dazu eingerichtet ist, bei einem Heizvorgang entstehende Schadstoffe zu binden, wobei bevorzugt das Filtermaterial Zeolith umfasst.

41. Evaporator nach einem der vorangegangenen Ansprüche, bei dem der Wirkstoff verkapselt ist, bevorzugt in Kugeln verkapselt ist, wobei bevorzugt eine Hülle der jeweiligen Kapseln dazu eingerichtet ist, Schadstoff zu binden und/oder zu filtern.

42. Evaporator nach einem der vorangegangenen Ansprüche, bei dem der Wirkstoff fest, flüssig, und/oder gasförmig ist und/oder bei dem der Wirkstoffvorläufer dazu eingerichtet ist, Wirkstoff in festem, flüssigen und/oder gasförmigem Zustand aufzunehmen.

43. Evaporator nach einem der vorangegangenen Ansprüche, bei dem der Wirkstoffvorläufer dazu eingerichtet ist, Wirkstoffe, die bevorzugt bei der Decarboxylierung von Cannabis entstehen, zu binden, und bei einer weiteren Aktivierung, bevorzugt bei einer Erhitzung, freizugeben.

44. Evaporator nach einem der vorangegangenen Ansprüche, bei dem der Wirkstoff gemahlene Cannabisblüten, und/oder eine Mischung aus gemahlene Cannabisblüten und Mehl, Wasser, Butter, Backpulver, und/oder Zucker umfasst.

45. Evaporator nach einem der vorangegangenen Ansprüche, **dadurch gekennzeichnet, dass** der Evaporator eine Kupplung aufweist, die dazu eingerichtet ist, ein Zusatzgerät am Evaporator anzudocken und/oder mit dem Evaporator zu verbinden, wobei bevorzugt das Zusatzgerät ein Tank, bevorzugt ein Tank zur Speicherung oder Bereitstellung gasförmiger Stoffe, eine Vakuumpumpe, und/oder ein Nassfilter ist oder aufweist.

46. Evaporator nach einem der Ansprüche 2 bis 45, der einen Flüssigkeitsspeicher aufweist, der an oder im Mundstück (Fig.1. 1) angeordnet ist und der als Nassfilter eingerichtet ist, wobei bevorzugt der Flüssigkeitsspeicher derart angeordnet ist, dass erzeugtes Aerosol und/oder Luft durch den Flüssigkeitsspeicher führbar ist.

47. Evaporator nach Anspruch 46, bei dem der Flüssigkeitsspeicher dazu eingerichtet ist, erzeugtes Aerosol zu reinigen und/oder mit Geschmack und/oder Geruch zu versetzen.

48. Evaporator nach einem der vorangegangenen Ansprüche, bei dem die Heizmuffe dazu eingerichtet ist, den Wirkstoffträger auf eine Temperatur von bis zu 300°C, bevorzugt auf bis zu 180°C, aufzuheizen.

## Claims

1. Evaporator for extracting and filtering active ingredients from biological materials by heating, wherein an active ingredient precursor (Fig. 1. 6) is arranged in a heating chamber (Fig. 1. 7), wherein the active ingredient precursor is surrounded by a heating sleeve (Fig. 4. 3) which is seated displaceably on the active ingredient precursor (Fig. 1. 6), wherein the length of the active substance ingredient is a multiple of the width of the heating sleeve (Fig. 4. 3), wherein the width of the heating sleeve is preferably approximately 5% to 25% of the length of the active ingredient precursor, such that a plurality, preferably approximately 4 to 15, subregions of the active ingredient precursor (Fig. 4. 2) can be heated individually, such that an active ingredient provided by the active ingredient precursor can be smoked at least in regions and/or an aerosol which comprises the active ingredient provided by the active ingredient precursor in regions can be generated, **characterized in that** the active ingredient precursor is configured in the form of a hollow cylinder, the outer walls of which are permeable to aerosols.

2. Evaporator according to claim 1, which has a mouthpiece which is designed to discharge and/or release an aerosol and/or air from the evaporator.

3. Evaporator according to one of the preceding claims, in which the heating chamber (Fig. 1. 7) (Fig. 4. 1) is double-walled with an inner wall and an outer wall, wherein a cavity is formed between the inner wall and the outer wall, wherein the heating chamber is preferably a double-walled cylinder, the inner diameter of which is preferably at least twice a diameter of the active ingredient precursor (Fig. 1. 1).

4. Evaporator according to claim 3, in which a vacuum exists in the cavity formed by the inner wall and the outer wall of the heating chamber or an active ingredient and/or coolant is located in the cavity.

5. Evaporator according to one of the preceding claims, in which an inner wall or the inner wall of the heating chamber (Fig. 1. 7) (Fig. 1. 7) has a nanocoating.

6. Evaporator according to one of claims 2 to 5, which has a filter in a region of the heating chamber (Fig. 1. 7) facing the mouthpiece, wherein the filter preferably comprises one or more of activated carbon, sponge and/or wet filter, statically charged metal wool, meltblown nonwoven or combinations thereof.

7. Evaporator according to one of claims 2 to 6, which has a superabsorber (Fig. 1. 10) in a region facing the mouthpiece, which superabsorber is designed to collect condensate, and/or in which, with reference to claim 6, the filter comprises a superabsorber or the superabsorber.

8. Evaporator according to one of the preceding claims, which has a line which is designed to conduct outside air from an air inlet opening (Fig. 1. 11) of the evaporator into the mouthpiece (Fig. 1. 1) of the evaporator and/or into the heating chamber, wherein the line is preferably arranged in the heating chamber and/or preferably in a cavity or the cavity formed by the inner wall and the outer wall of the heating chamber.

9. Evaporator according to one of the preceding claims, in which an outer wall of the evaporator is coated with thermochromatic lacquer, which is designed to change its colour as a function of temperature.

10. Evaporator according to one of the preceding claims, **characterized in that** the evaporator has a housing (Fig. 1. 18) which consists of a magnetic wire grid or has a magnetic wire grid, wherein the magnetic wire grid is coated in a planar manner with plastic which, when current flows through wires of the wire grid, insulates the current-conducting wires from one another.

11. Evaporator according to claim 10, which has at least one switching element arranged on or at a surface of the housing, wherein the switching element is preferably inductively coupled to at least one wire and/or the magnetic wire grid.

12. Evaporator according to one of the preceding claims, in which the active ingredient precursor (Fig. 1. 6) forms a receptacle, preferably a cylindrical receptacle, and/or is dimensioned such that it has the at least one length of 8 cm, preferably at least 8.4 cm, and a diameter of at least 0.6 cm, preferably at least 0.8 cm, wherein the active ingredient precursor is preferably dimensioned such that it completely receives a cigarette.

13. Evaporator according to one of the preceding claims, in which the active ingredient precursor and/or the receptacle has a cylindrical wire basket which is preferably divided into at least five zones, wherein the zones preferably have no electrical contact with one another and/or are electrically insulated from one another.

14. Evaporator according to claim 13, **characterized in that** the heating sleeve (Fig. 4. 3) is movable and at least partially encloses the active ingredient precursor (Fig. 4. 2), and wherein the heating sleeve has sliding contacts (Fig. 5. 11) which are designed to supply current to and/or heat the individual zones of the wire basket, preferably in order to heat active ingredients received in a zone and/or preferably in order to form an aerosol.

15. Evaporator according to one of the preceding claims, which has a sliding element which is arranged outside the heating chamber (Fig. 1. 7) (Fig. 4. 1) and which is coupled to the heating sleeve, so that a movement of the sliding element can be transmitted to the heating sleeve and/or vice versa.

16. Evaporator according to claim 15, in which the sliding element (Fig. 4. 12) is elastically connected to the heating sleeve (Fig. 4. 3), wherein the elastic connection is or preferably has a silicone tube (Fig. 4. 7) and/or electrically conductive material.

17. Evaporator according to claim 15 or 16, in which the sliding element has an active ingredient chamber, in which active ingredient can be received, and the silicone tube is preferably designed to transport active ingredient from the active ingredient chamber into the heating chamber (Fig. 1. 7) and/or the heating sleeve.

18. Evaporator according to claim 17, in which the active ingredient chamber can be reduced in size by pressure with the aid of a membrane (Fig. 4. 14), so that active ingredient can be fed into the silicone tube, the heating sleeve and/or the heating chamber.

19. Evaporator according to claim 17 or 18, in which the evaporator has a deflection roller which is designed to transport active ingredient into the heating chamber (Fig. 1. 7) and/or the heating sleeve, wherein the deflection roller (Fig. 4. 10) preferably has convex bulges and/or is preferably designed as a peristaltic pump.

20. Evaporator according to one of the preceding claims, in which the heating sleeve (Fig. 4. 3) has a housing chamber wall which has a filter material through which air and/or aerosol formed in the heating sleeve, and/or aerosol formed in the region of the active ingredient carrier enclosed by the heating sleeve, can be diffused.

21. Evaporator according to claim 20, **characterized in that** the housing chamber wall and/or the filter material has pores which are designed to change their size as a function of temperature.

22. Evaporator according to one of the preceding claims, **characterized in that** a housing or the housing (Fig. 1. 18) of the heating sleeve (Fig. 4. 3) consists of a material or has a material which filters or binds harmful substances, wherein the material preferably comprises one or more zeolites, preferably inactivated zeolites with a grain size of 0.1 to 2 mm, marble, activated carbon, clay, ash and/or a mixture thereof.

23. Evaporator according to one of the preceding claims, in which the heating sleeve (Fig. 4. 3) has a seal, preferably a sealing lip, which seals the heating sleeve and/or which is designed to compress and/or reduce a region of the heating chamber, preferably a region of the heating chamber arranged between seal and mouthpiece, when the heating sleeve is moved.

24. Evaporator according to one of the preceding claims, in which the heating sleeve (Fig. 4. 3) has a seal or the seal, preferably a sealing lip or the sealing lip, which is designed to convey air, active ingredient and/or aerosol, preferably through a housing or the porous housing of the heating sleeve, when the heating sleeve is moved.

25. Evaporator according to one of the preceding claims, wherein the heating sleeve has a heating coil.

26. Evaporator according to claim 25, in which the heating coil has a memory alloy or consists of a memory alloy, wherein the heating coil and/or the memory alloy is designed to expand on heating and to contract on cooling.

27. Evaporator according to claim 25 or 26, wherein the heating sleeve has a front sealing disc and a rear sealing disc, between which the heating coil is arranged and which are connected to the heating coil, wherein the front and/or the rear sealing disc is/are designed to change their diameter.

28. Evaporator according to claim 27, in which the front sealing disc and/or the rear sealing disc each have a first part and a second part, wherein the first part and the second part are preferably substantially semicircular and/or semi-annular, wherein the respective first part is connected to the respective second part via a spring, so that the diameter of the respective sealing disc can be changed, wherein the spring preferably comprises a memory metal or consists of such a metal, so that the spring preferably expands or contracts as a function of temperature.

29. Evaporator according to claim 27 or 28, in which the front sealing disc and/or the rear sealing disc can be folded, preferably can be folded in and/or through an angle of up to 130°, so that the diameter of the respective sealing disc can be changed by folding between an unfolded position and a folded position, wherein the front sealing disc and the rear sealing disc are preferably designed to be folded as a function of temperature.

30. Evaporator according to claim 29, in which the front sealing disc and/or the rear sealing disc is clamped to and/or contacts an inner wall or the inner wall of the heating chamber when the respective sealing disc is unfolded, and the respective sealing disc is displaceable and/or movable in the heating chamber when the respective sealing disc is folded.

31. Evaporator according to one of the preceding claims, which is designed to generate aerosol in the heating chamber (Fig. 1. 7) by mixing active ingredient with air, preferably outside air.

32. Evaporator according to one of claims 2 to 31, which is designed to generate aerosol by mixing active ingredient with air, preferably outside air, in the mouthpiece (Fig. 1. 1).

33. Evaporator according to one of claims 2 to 32, in which the mouthpiece comprises a flavouring substance and/or fragrance, wherein the flavouring substance and/or fragrance is preferably encapsulated and/or the mouthpiece preferably has a porous material through which flavouring substance and/or fragrance can pass.

34. Evaporator according to claim 33, in which the flavouring substance and/or fragrance is encapsulated in a cellulose polymer, preferably a cellulose polymer of wood pulp.

35. Evaporator according to claim 33 or 34, in which the mouthpiece has a reservoir for the flavouring substance and/or fragrance and/or in which the mouthpiece is designed to add and/or admix the flavouring substance and/or fragrance to aerosol and/or air conveyed through the mouthpiece.

36. Evaporator according to one of claims 2 to 35, which has a flap (Fig. 1. 15) which is arranged in the mouthpiece (Fig. 1. 1) and which is designed to react to suction, so that when the flap reacts to suction through the mouthpiece, a control element (Fig. 1. 17) and/or the heating sleeve is activated and/or an aerosol or the aerosol is formed in the heating chamber and/or in the mouthpiece.

37. Evaporator according to one of claims 2 to 36, in which the mouthpiece (Fig. 1. 1) has a nozzle through which aerosol and/or air can be discharged from the mouthpiece.

38. Evaporator according to one of the preceding claims, in which the active ingredient precursor (Fig. 1. 6) is designed to receive active ingredient, and/or in which the active ingredient precursor has a receptacle and active ingredient received in the receptacle.

39. Evaporator according to claim 38, in which different active ingredients are each received in the receptacle in at least two different regions of the active ingredient precursor.

40. Evaporator according to one of the preceding claims, in which the active ingredient precursor has a filter material and/or consists of a filter material which is designed to bind harmful substances arising during a heating process, wherein the filter material preferably comprises zeolite.

41. Evaporator according to one of the preceding claims, in which the active ingredient is encapsulated, preferably encapsulated in spheres, wherein a casing of the respective capsules is preferably designed to bind and/or filter harmful substance.

42. Evaporator according to one of the preceding claims, in which the active ingredient is solid, liquid and/or gaseous and/or in which the active ingredient precursor is designed to receive active ingredient in the solid, liquid and/or gaseous state.

43. Evaporator according to one of the preceding claims, in which the active ingredient precursor is designed to bind active ingredients which preferably arise during the decarboxylation of cannabis, and release them upon further activation, preferably upon heating.

44. Evaporator according to one of the preceding claims, in which the active ingredient comprises ground cannabis blossoms, and/or a mixture of ground cannabis blossoms and flour, water, butter, baking powder, and/or sugar.

45. Evaporator according to one of the preceding claims, **characterized in that** the evaporator has a coupling which is designed to dock an additional device on the evaporator and/or to connect it to the evaporator, wherein the additional device preferably is or has a tank, preferably a tank for storing or providing gaseous substances, a vacuum pump, and/or a wet filter.

46. Evaporator according to one of claims 2 to 45, which has a liquid reservoir which is arranged on or in the mouthpiece (Fig. 1. 1) and which is designed as a wet filter, wherein the liquid reservoir is preferably arranged such that generated aerosol and/or air can be guided through the liquid reservoir.

47. Evaporator according to claim 46, in which the liquid reservoir is designed to clean generated aerosol and/or to treat it with taste and/or smell.

48. Evaporator according to one of the preceding claims, in which the heating sleeve is designed to heat the active ingredient carrier to a temperature of up to 300°C, preferably to up to 180°C.

## Revendications

1. Évaporateur pour extraire et filtrer par chauffage des substances actives à partir de matériaux biologiques, dans lequel un précurseur de substance active (fig. 1. 6) est agencé dans une chambre de chauffage (fig. 1. 7), le précurseur de substance active étant entouré d'un manchon chauffant (fig. 4. 3) qui est placé de manière coulissante sur le précurseur de substance active (fig. 1. 6), la longueur du précurseur de substance active étant un multiple de la largeur du manchon chauffant (fig. 4. 3), la largeur du manchon chauffant représentant de préférence environ 5 % à 25 % de la longueur du précurseur de substance active, de sorte qu'une pluralité de zones partielles du précurseur de substance active (fig. 4. 2), de préférence environ 4 à 15, peuvent être chauffées individuellement, de sorte que la substance active fournie par le précurseur de substance active peut être fumée au moins par zones et/ou qu'un aérosol comprenant, par zones, la substance active fournie par le précurseur de substance active peut être généré, **caractérisé en ce que** le précurseur de substance active est conçu sous la forme d'un cylindre creux dont les parois extérieures sont perméables aux aérosols.

2. Évaporateur selon la revendication 1, qui présente un embout buccal qui est conçu pour évacuer et/ou délivrer un aérosol et/ou de l'air partant de l'évaporateur.

3. Évaporateur selon l'une des revendications précédentes, dans lequel la chambre de chauffage (fig. 1. 7) (fig. 4. 1) est à double paroi avec une paroi intérieure et une paroi extérieure, un espace creux étant formé entre la paroi intérieure et la paroi extérieure, la chambre de chauffage étant de préférence un cylindre à double paroi dont le diamètre intérieur est de préférence au moins le double du diamètre du précurseur de substance active (fig. 1. 1).

4. Évaporateur selon la revendication 3, dans lequel il existe un vide dans la cavité formée par la paroi intérieure et la paroi extérieure de la chambre de chauffage ou dans lequel une substance active et/ou un réfrigérant se trouve dans la cavité.

5. Évaporateur selon l'une des revendications précédentes, dans lequel une ou la paroi intérieure de la chambre de chauffage (fig. 1. 7) (fig. 1. 7) présente un nanorevêtement.

6. Évaporateur selon l'une des revendications 2 à 5, qui présente un filtre dans une zone de la chambre de chauffage (fig. 1. 7) tournée vers l'embout buccal, le filtre comprenant de préférence un ou plusieurs des éléments suivants : charbon actif, éponge et/ou filtre humide, laine métallique à charge statique, non-tissé meltblown ou combinaison de ces matériaux.

7. Évaporateur selon l'une des revendications 2 à 6, qui présente, dans une zone tournée vers l'embout buccal, un superabsorbant (fig. 1. 10) qui est agencé pour collecter le condensat et/ou dans lequel, en référence à la revendication 6, le filtre comprend un ou le superabsorbant.

8. Évaporateur selon l'une des revendications précédentes, qui présente un conduit conçu pour acheminer l'air extérieur depuis une ouverture d'entrée d'air (fig. 1. 11) de l'évaporateur vers l'embout buccal (fig. 1. 1) de l'évaporateur et/ou vers la chambre de chauffage, le conduit étant de préférence agencé dans la chambre de chauffage et/ou de préférence dans une cavité formée par la paroi intérieure et la paroi extérieure de la chambre de chauffage.

9. Évaporateur selon l'une des revendications précédentes, dans lequel une paroi extérieure de l'évaporateur est recouverte d'une peinture thermochromatique conçue pour changer de couleur en fonction de la température.

10. Évaporateur selon l'une des revendications précédentes, **caractérisé en ce que** l'évaporateur présente un boîtier (fig. 1. 18) constitué d'un treillis métallique magnétique ou présentant un treillis métallique magnétique, le treillis métallique magnétique étant recouvert d'une couche de matière plastique qui, lorsque le courant circule dans les fils du treillis métallique, isole les fils conducteurs les uns des autres.

11. Évaporateur selon la revendication 10, qui présente au moins un élément de commutation agencé sur ou à proximité d'une surface du boîtier, l'élément de commutation étant de préférence couplé de manière inductive à au moins un fil et/ou à la grille magnétique en fil métallique.

12. Évaporateur selon l'une des revendications précédentes, dans lequel le précurseur de substance active (fig. 1. 6) forme un réceptacle, de préférence un réceptacle cylindrique, et/ou est dimensionné de telle sorte qu'il présente une longueur d'au moins 8 cm, de préférence d'au moins 8,4 cm, et un diamètre d'au moins 0,6 cm, de préférence d'au moins 0,8 cm, le précurseur de substance active étant de préférence dimensionné de manière à pouvoir recevoir une cigarette entière.

13. Évaporateur selon l'une des revendications précédentes, dans lequel le précurseur de substance active et/ou le réceptacle présente un panier métallique cylindrique, qui est de préférence divisé en au moins cinq zones, les zones n'étant de préférence pas en contact électrique les unes avec les autres et/ou étant isolées électriquement les unes des autres.

14. Évaporateur selon la revendication 13, **caractérisé en ce que** le manchon chauffant (fig. 4. 3) est mobile et entoure au moins partiellement le précurseur de substance active (fig. 4. 2), et le manchon chauffant présentant des contacts à frottement (fig. 5. 11) qui sont conçus pour alimenter en courant et/ou chauffer les différentes zones du panier métallique, de préférence pour chauffer les substances actives logées dans une zone et/ou pour former de préférence un aérosol.

15. Évaporateur selon l'une des revendications précédentes, qui présente un élément coulissant agencé à l'extérieur de la chambre de chauffage (fig. 1. 7) (fig. 4. 1) et qui est couplé au manchon chauffant, de sorte qu'un mouvement de l'élément coulissant peut être transmis au manchon chauffant et/ou inversement.

16. Évaporateur selon la revendication 15, dans lequel l'élément coulissant (fig. 4. 12) est relié de manière élastique au manchon chauffant (fig. 4. 3), la liaison élastique étant de préférence un tuyau en silicone (fig. 4. 7) et/ou un matériau électriquement conducteur ou présentant un tel matériau.

17. Évaporateur selon la revendication 15 ou 16, dans lequel l'élément coulissant présente une chambre de substance active dans laquelle peut être introduite une substance active et dans lequel, de préférence, le tuyau en silicone est conçu pour transporter la substance active de la chambre de substance active à la chambre de chauffage (fig. 1. 7) et/ou au manchon chauffant.

18. Évaporateur selon la revendication 17, dans lequel la chambre de substance active peut être réduite par pression à l'aide d'une membrane (fig. 4. 14), de sorte que la substance active peut être introduite dans le tuyau en silicone, le manchon chauffant et/ou la chambre de chauffage.

19. Évaporateur selon la revendication 17 ou 18, dans lequel l'évaporateur présente une poulie de renvoi qui est conçue pour transporter la substance active dans la chambre de chauffage (fig. 1.7) et/ou le manchon chauffant, la poulie de renvoi (fig. 4.10) présentant de préférence des renflements convexes et/ou étant de préférence conçue comme une pompe à tuyau.

20. Évaporateur selon l'une des revendications précédentes, dans lequel le manchon chauffant (fig. 4.3) présente une paroi de chambre de boîtier qui présente un matériau filtrant à travers lequel l'air et/ou l'aérosol formé dans le manchon chauffant et/ou l'aérosol formé dans la zone du support de substance active entourée par le manchon chauffant peuvent se diffuser.

21. Évaporateur selon la revendication 20, **caractérisé en ce que** la paroi de la chambre du boîtier et/ou le matériau filtrant présentent des pores qui sont conçus pour modifier leur taille en fonction de la température.

22. Évaporateur selon l'une des revendications précédentes, **caractérisé en ce qu'**un ou le boîtier (fig. 1. 18) du manchon chauffant (fig. 4. 3) est constitué d'un matériau ou présente un matériau qui filtre ou retient les substances nocives, le matériau comprenant de préférence une ou plusieurs zéolithes, de préférence des zéolithes inactivées d'une granulométrie de 0,1 à 2 mm, du marbre, du charbon actif, de l'argile, des cendres et/ou un mélange de ceux-ci.

23. Évaporateur selon l'une des revendications précédentes, dans lequel le manchon chauffant (fig. 4. 3) présente un joint d'étanchéité, de préférence une lèvre d'étanchéité, qui assure l'étanchéité du manchon chauffant et/ou qui est conçu de manière à comprimer et/ou à réduire une zone de la chambre de chauffage, de préférence une zone de la chambre de chauffage située entre le joint d'étanchéité et l'embout buccal, lorsque le manchon chauffant est déplacé.

24. Évaporateur selon l'une des revendications précédentes, dans lequel le manchon chauffant (fig. 4. 3) présente un ou plusieurs joints d'étanchéité, de préférence une ou plusieurs lèvres d'étanchéité, qui sont conçus de telle sorte que, lorsque le manchon chauffant est déplacé, de l'air, une substance active et/ou un aérosol sont transportés, de préférence à travers un ou plusieurs boîtiers poreux du manchon chauffant.

25. Évaporateur selon l'une des revendications précédentes, dans lequel le manchon chauffant présente un serpentin chauffant.

26. Évaporateur selon la revendication 25, dans lequel le serpentin chauffant présente un alliage à mémoire de forme ou est constitué d'un alliage à mémoire de forme, le serpentin chauffant et/ou l'alliage à mémoire de forme étant conçu pour se dilater lors du chauffage et se contracter lors du refroidissement.

27. Évaporateur selon la revendication 25 ou 26, dans lequel le manchon chauffant présente une rondelle d'étanchéité avant et une rondelle d'étanchéité arrière, entre lesquelles est agencé le serpentin chauffant et qui sont reliées au serpentin chauffant, la rondelle d'étanchéité avant et/ou la rondelle d'étanchéité arrière étant conçues pour modifier leur diamètre.

28. Évaporateur selon la revendication 27, dans lequel la rondelle d'étanchéité avant et/ou la rondelle d'étanchéité arrière présentent chacune une première partie et une deuxième partie, la première partie et la deuxième partie étant de préférence sensiblement semi-circulaires et/ou semi-annulaires, la première partie respective étant reliée à la deuxième partie respective par un ressort, de sorte que le diamètre de chaque rondelle d'étanchéité peut être modifié, le ressort comprenant de préférence un métal à mémoire de forme ou étant constitué d'un tel métal, de sorte que le ressort se dilate ou se contracte de préférence en fonction de la température.

29. Évaporateur selon la revendication 27 ou 28, dans lequel la rondelle d'étanchéité avant et/ou la rondelle d'étanchéité arrière est pliable, de préférence dans et/ou autour d'un angle allant jusqu'à 130°, de sorte que le diamètre de chaque rondelle d'étanchéité peut être modifié par pliage entre une position non pliée et une position pliée, la rondelle d'étanchéité avant et la rondelle d'étanchéité arrière étant de préférence conçues pour être pliables en fonction de la température.

30. Évaporateur selon la revendication 29, dans lequel la rondelle d'étanchéité avant et/ou la rondelle d'étanchéité arrière sont coincées avec une ou la paroi intérieure de la chambre de chauffage et/ou entrent en contact avec celle-ci lorsque la rondelle d'étanchéité respective n'est pas pliée, et dans lequel chaque rondelle d'étanchéité est coulissante et/ou mobile dans la chambre de chauffage lorsque la rondelle d'étanchéité correspondante est pliée.

31. Évaporateur selon l'une des revendications précédentes, qui est conçu pour produire un aérosol dans la chambre de chauffage (fig. 1. 7) en mélangeant une substance active avec de l'air, de préférence de l'air extérieur.

32. Évaporateur selon l'une des revendications 2 à 31, qui est conçu pour produire un aérosol par mélange d'une substance active avec de l'air, de préférence de l'air extérieur, dans l'embout buccal (fig. 1. 1) (fig. 1. 1).

33. Évaporateur selon l'une des revendications 2 à 32, dans lequel l'embout buccal contient un arôme et/ou un parfum encapsulé et/ou, de préférence, l'embout buccal présente un matériau poreux à travers lequel l'arôme et/ou le parfum peuvent passer.

34. Évaporateur selon la revendication 33, dans lequel l'arôme et/ou le parfum sont encapsulés dans un polymère de cellulose, de préférence un polymère de cellulose issu de la cellulose de bois.

35. Évaporateur selon l'une des revendications 33 ou 34, dans lequel l'embout buccal présente un réservoir pour l'arôme et/ou le parfum et/ou dans lequel l'embout buccal est agencé pour ajouter et/ou mélanger l'arôme et/ou le parfum à l'aérosol et/ou à l'air transporté à travers l'embout buccal.

36. Évaporateur selon l'une des revendications 2 à 35, qui présente un clapet (fig. 1. 1) agencé dans l'embout buccal (fig. 1. 15) qui est conçu pour réagir à une aspiration, de sorte que lorsque le clapet réagit à une aspiration à travers l'embout buccal, un élément de commande (fig. 1. 17) et/ou le manchon chauffant est activé et/ou un ou plusieurs aérosols sont formés dans la chambre de chauffage et/ou dans l'embout buccal.

37. Évaporateur selon l'une des revendications 2 à 36, dans lequel l'embout buccal (fig. 1. 1) présente une buse à travers laquelle l'aérosol et/ou l'air peuvent être évacués de l'embout buccal.

38. Évaporateur selon l'une des revendications précédentes, dans lequel le précurseur de substance active (fig. 1. 6) est conçu pour recevoir la substance active et/ou dans lequel le précurseur de substance active présente un réceptacle et la substance active reçue dans le réceptacle.

39. Évaporateur selon la revendication 38, dans lequel différentes substances actives sont absorbées dans le réceptacle dans au moins deux zones différentes du précurseur de substance active.

40. Évaporateur selon l'une des revendications précédentes, dans lequel le précurseur de substance active présente un matériau filtrant et/ou est constitué d'un matériau filtrant qui est conçu pour fixer les substances nocives produites lors d'un processus de chauffage, le matériau filtrant comprenant de préférence de la zéolithe.

41. Évaporateur selon l'une des revendications précédentes, dans lequel la substance active est encapsulée, de préférence dans des capsules, l'enveloppe des capsules respectives étant de préférence conçue pour fixer et/ou filtrer les substances nocives.

42. Évaporateur selon l'une des revendications précédentes, dans lequel la substance active est solide, liquide et/ou gazeuse et/ou dans lequel le précurseur du substance active est conçu pour absorber la substance active à l'état solide, liquide et/ou gazeux.

43. Évaporateur selon l'une des revendications précédentes, dans lequel le précurseur de substance active est conçu pour fixer les substances actives qui se forment de préférence lors de la décarboxylation du cannabis et pour les libérer lors d'une activation supplémentaire, de préférence lors d'un chauffage.

44. Évaporateur selon l'une des revendications précédentes, dans lequel la substance active comprend des fleurs de cannabis moulues et/ou un mélange de fleurs de cannabis moulues et de farine, d'eau, de beurre, de levure chimique et/ou de sucre.

45. Évaporateur selon l'une des revendications précédentes, **caractérisé en ce que** l'évaporateur présente un raccordement qui est conçu pour fixer un appareil supplémentaire à l'évaporateur et/ou pour le relier à l'évaporateur, l'appareil supplémentaire étant de préférence un réservoir, de préférence un réservoir pour le stockage ou la mise à disposition de substances gazeuses, une pompe à vide et/ou un filtre humide.

46. Évaporateur selon l'une des revendications 2 à 45, qui présente un réservoir de liquide agencé sur ou dans l'embout buccal (fig. 1. 1) et qui est conçu comme un filtre humide, le réservoir de liquide étant de préférence agencé de telle sorte que l'aérosol et/ou l'air produits puissent passer à travers le réservoir de liquide.

47. Évaporateur selon la revendication 46, dans lequel le réservoir de liquide est conçu pour purifier l'aérosol produit et/ou lui ajouter un arôme et/ou un parfum.

48. Évaporateur selon l'une des revendications précédentes, dans lequel le manchon chauffant est conçu pour chauffer le support de substance active à une température pouvant atteindre 300°C, de préférence 180°C.
